(19) Europäisches Patentamt
European Patent Office
Office européen des brevets

(11) EP 2 726 475 B1

(12) **EUROPEAN PATENT SPECIFICATION**

(45) Date of publication and mention of the grant of the patent:
**25.10.2017 Bulletin 2017/43**

(21) Application number: **12725228.6**

(22) Date of filing: **23.05.2012**

(51) Int Cl.:
***C07D 401/12*** *(2006.01)* ***A61K 31/4725*** *(2006.01)*
***A61P 31/14*** *(2006.01)*

(86) International application number:
**PCT/US2012/039090**

(87) International publication number:
**WO 2012/166459 (06.12.2012 Gazette 2012/49)**

(54) **TRIPEPTIDES INCORPORATING DEUTERIUM AS INHIBITORS OF HEPATITIS C VIRUS**

DEUTERIUM ENTHALTENDE TRIPEPTIDE ALS INHIBITOREN DES HEPATITIS-C-VIRUS

TRIPEPTIDES CONTENANT DU DEUTERIUM COMME INHIBITEURS DU VIRUS DE L'HEPATITE C

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2011 US 201161490665 P**

(43) Date of publication of application:
**07.05.2014 Bulletin 2014/19**

(73) Proprietor: **Bristol-Myers Squibb Company**
**Princeton, NJ 08543-4000 (US)**

(72) Inventors:
- **SUN, Li-Qiang**
  **Wallingford, Connecticut 06492 (US)**
- **SCOLA, Paul Michael**
  **Wallingford, Connecticut 06492 (US)**

(74) Representative: **Reitstötter Kinzebach**
**Patentanwälte**
**Sternwartstrasse 4**
**81679 München (DE)**

(56) References cited:
**WO-A1-2009/129109 WO-A1-2011/038283**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 726 475 B1

## Description

CROSS-REFERENCE TO RELATED APPLICATIONS

This application claims the benefit of U.S. Provisional Application Serial Number 61/490,665 filed May 27, 2011.

The present disclosure relates to deuterated *N*-(*tert*-butoxycarbonyl)-3-methyl-L-valyl-(4*R*)-*N*-((1*R*,2*S*)-1{[(cyclopropylsulfonyl)amino]carbonyl}-2-vinylcyclopropyl) -4-[(4-methoxy-7-chloroisoquinolin-1-yl)oxy]-L-prolinamide compounds, their pharmaceutical compositions, process thereof, and methods of use. The compounds possess the ability to inhibit NS3 protease (also referred to herein as "serine protease" and are useful in the treatment of hepatitis C virus.

HCV is a major human pathogen, infecting an estimated 170 million persons worldwide - roughly five times the number infected by human immunodeficiency virus type 1. A substantial fraction of these HCV infected individuals develop serious progressive liver disease, including cirrhosis and hepatocellular carcinoma.

Presently, the most effective HCV therapy employs a combination of alpha-interferon and ribavirin, leading to sustained efficacy in 40% of patients. Recent clinical results demonstrate that pegylated alpha-interferon is superior to unmodified alpha-interferon as monotherapy. However, even with experimental therapeutic regimens involving combinations of pegylated alpha-interferon and ribavirin, a substantial fraction of patients do not have a sustained reduction in viral load. Thus, there is a clear and unmet need to develop effective therapeutics for treatment of HCV infection.

HCV is a positive-stranded RNA virus. Based on a comparison of the deduced amino acid sequence and the extensive similarity in the 5' untranslated region, HCV has been classified as a separate genus in the Flaviviridae family. All members of the Flaviviridae family have enveloped virions that contain a positive stranded RNA genome encoding all known virus-specific proteins *via* translation of a single, uninterrupted, open reading frame.

Considerable heterogeneity is found within the nucleotide and encoded amino acid sequence throughout the HCV genome. Six major genotypes have been characterized, and more than 50 subtypes have been described. The major genotypes of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non- structural (NS) proteins. In the case of HCV, the generation of mature non-structural proteins (NS2, NS3, NS4A, NS4B, NS5A, and NS5B) is effected by two viral proteases. The first one cleaves at the NS2-NS3 junction; the second one is a serine protease contained within the *N*-terminal region of NS3 and mediates all the subsequent cleavages downstream of NS3, both in cis, at the NS3-NS4A cleavage site, and in trans, for the remaining NS4A- NS4B, NS4B-NS5A, NS5A-NS5B sites. The NS4A protein appears to serve multiple functions, acting as a co-factor for the NS3 protease and possibly assisting in the membrane localization of NS3 and other viral replicase components. The complex formation of the NS3 protein with NS4A is essential for efficient polyprotein processing, enhancing the proteolytic cleavage at all of the sites. The NS3 protein also exhibits nucleoside triphosphatase and RNA helicase activities. NS5B is a RNA-dependent RNA polymerase that is involved in the replication of HCV.

The present disclosure provides peptide compounds that can inhibit the functioning of the NS3 protease, e.g., in combination with the NS4A protease.

In its first aspect the present disclosure provides a compound of Formula (I)

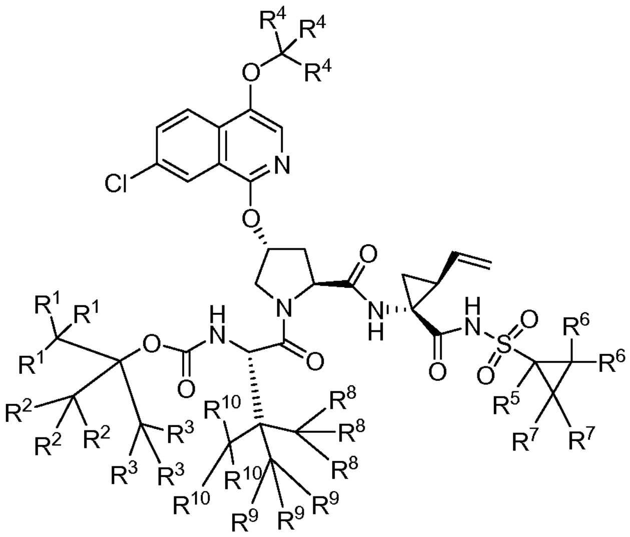

(I),

or a pharmaceutically acceptable salt thereof, wherein

$R^1$, $R^2$, $R^3$, $R^4$, $R^5$, $R^6$, $R^7$, $R^8$, $R^9$, and $R^{10}$ are independently selected from hydrogen and deuterium; provided that at least one is other than hydrogen.

In a first embodiment of the first aspect the present disclosure provides a compound of Formula (I), or a pharmaceutically acceptable salt thereof, wherein each $R^1$ is deuterium. In a second embodiment of the first aspect each $R^2$ is deuterium. In a third embodiment of the first aspect each $R^3$ is deuterium.

In a fourth embodiment of the first aspect the present disclosure provides a compound of Formula (I), or a pharmaceutically acceptable salt thereof, wherein each $R^4$ is deuterium.

In a fifth embodiment of the first aspect the present disclosure provides a compound of Formula (I), or a pharmaceutically acceptable salt thereof, wherein $R^5$ is deuterium.

In a sixth embodiment of the first aspect the present disclosure provides a compound of Formula (I), or a pharmaceutically acceptable salt thereof, wherein each $R^6$ is deuterium. In a seventh embodiment each $R^7$ is deuterium.

In an eighth embodiment of the first aspect the present disclosure provides a compound of Formula (I), or a pharmaceutically acceptable salt thereof, wherein each $R^8$ is deuterium. In a ninth embodiment each $R^9$ is deuterium. In a tenth embodiment each $R^{10}$ is deuterium.

In a second aspect the present disclosure provides a composition comprising the compound of Formula (I), or a pharmaceutically acceptable salt thereof, and a pharmaceutically acceptable carrier. In a first embodiment of the second aspect the composition further comprises at least one additional compound having anti-HCV activity. In a second embodiment of the second aspect at least one of the additional compounds is an interferon or a ribavirin. In a third embodiment of the second aspect the interferon is selected from interferon alpha 2B, pegylated interferon alpha, consensus interferon, interferon alpha 2A, and lymphoblastiod interferon tau.

In a fourth embodiment of the second aspect the present disclosure provides a composition comprising the compound of Formula (I), or a pharmaceutically acceptable salt thereof, a pharmaceutically acceptable carrier, and at least one additional compound having anti-HCV activity, wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

In a fifth embodiment of the second aspect the present disclosure provides a composition comprising the compound of Formula (I), or a pharmaceutically acceptable salt thereof, a pharmaceutically acceptable carrier, and at least one additional compound having anti-HCV activity, wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

In a third aspect the present disclosure provides a composition comprising a compound of Formula (I), or a pharmaceutically acceptable salt thereof, one, two, three, four, or five additional compounds having anti-HCV activity, and a pharmaceutically acceptable carrier. In a first embodiment of the third aspect the compsition comprises three or four additional compounds having anti-HCV activity. In a second embodiment of the third aspect the composition comprises one or two additional compounds having anti-HCV activity. The present disclosure describes a method of treating

an HCV infection in a patient, comprising administering to the patient a therapeutically effective amount of Formula (I), or a pharmaceutically acceptable salt thereof. In a first embodiment of the fourth aspect the method further comprises administering at least one additional compound having anti-HCV activity prior to, after, or simultaneously with the compound of Formula (I), or a pharmaceutically acceptable salt thereof. In a second embodiment of the fourth aspect at least one of the additional compounds is an interferon or a ribavirin. In a third embodiment of the fourth aspect the interferon is selected from interferon alpha 2B, pegylated interferon alpha, consensus interferon, interferon alpha 2A, and lymphoblastiod interferon tau. The present disclosure also describes a method of treating an HCV infection in a patient, comprising administering to the patient a therapeutically effective amount of Formula (I), or a pharmaceutically acceptable salt thereof, and at least one additional compound having anti-HCV activity prior to, after, or simultaneously with the compound of Formula (I), or a pharmaceutically acceptable salt thereof, wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine. Furthermore, the present disclosure describes a method of treating an HCV infection in a patient, comprising administering to the patient a therapeutically effective amount of Formula (I), or a pharmaceutically acceptable salt thereof, and at least one additional compound having anti-HCV activity prior to, after, or simultaneously with the compound of Formula (I), or a pharmaceutically acceptable salt thereof, wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection. And lastly, the present disclosure describes a method of treating an HCV infection in a patient, comprising administering to the patient a therapeutically effective amount of a compound of formula (I), or a pharmaceutically acceptable salt thereof and one, two, three, four, or five additional compounds having anti-HCV activity prior to, after, or simultaneously with the compound of formula (I), or a pharmaceutically acceptable salt thereof or administering three or four additional compounds having anti-HCV activity or administering one or two additional compounds having anti-HCV activity.

Other aspects of the present disclosure may include suitable combinations of embodiments disclosed herein.

Yet other aspects and embodiments may be found in the description provided herein.

It should be understood that the compounds encompassed by the present disclosure are those that are suitably stable for use as pharmaceutical agent.

It is intended that the definition of any substituent or variable at a particular location in a molecule be independent of its definitions elsewhere in that molecule. For example, each of the three $R^4$ groups may be the same or different.

As used herein, the singular forms "a", "an", and "the" include plural reference unless the context clearly dictates otherwise.

It is understood that deuterium incorporation in Formulas or schemes may be indicated by use of the symbols "D", "d" or "$^2$H".

The compounds of the present disclosure can exist as pharmaceutically acceptable salts. The term "pharmaceutically acceptable salt," as used herein, represents salts or zwitterionic forms of the compounds of the present disclosure which are water or oil-soluble or dispersible, which are, within the scope of sound medical judgment, suitable for use in contact with the tissues of patients without excessive toxicity, irritation, allergic response, or other problem or complication commensurate with a reasonable benefit/risk ratio, and are effective for their intended use. The salts can be prepared during the final isolation and purification of the compounds or separately by reacting a suitable basic functionality with a suitable acid. Representative acid addition salts include acetate, adipate, alginate, citrate, aspartate, benzoate, benzenesulfonate, bisulfate, butyrate, camphorate, camphorsulfonate; digluconate, glycerophosphate, hemisulfate, heptanoate, hexanoate, formate, fumarate, hydrochloride, hydrobromide, hydroiodide, 2-hydroxyethanesulfonate, lactate, maleate, mesitylenesulfonate, methanesulfonate, naphthylenesulfonate, nicotinate, 2-naphthalenesulfonate, oxalate, palmoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Examples of acids which can be employed to form pharmaceutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting an acidic group with a suitable base such as the hydroxide, carbonate, or bicarbonate of a metal cation or with ammonia or an organic primary, secondary, or tertiary amine. The cations of pharmaceutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, *N,N*-dimethylaniline, *N*-methylpiperidine, *N*-methylmorpholine, dicyclohexylamine, procaine, dibenzylamine, *N,N*-dibenzylphenethylamine, and *N,N'*-dibenzylethylenediamine. Other representative organic amines useful for the formation of base addition salts include ethylenediamine, ethanolamine, diethanolamine, piperidine, and piperazine.

As used herein, the term "anti-HCV activity" means the compound is effective to treat the HCV virus.

The term "compounds of the disclosure", and equivalent expressions, are meant to embrace compounds of formula (I), and pharmaceutically acceptable enantiomers, diastereomers, and salts thereof. Similarly, references to intermediates, are meant to embrace their salts where the context so permits.

The term "patient" includes both human and other mammals.

The term "pharmaceutical composition" means a composition comprising a compound of the disclosure in combination with at least one additional pharmaceutical carrier, i.e., adjuvant, excipient or vehicle, such as diluents, preserving agents, fillers, flow regulating agents, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms. Ingredients listed in Remington's Pharmaceutical Sciences, 18th ed., Mack Publishing Company, Easton, PA (1999) for example, may be used.

The phrase "pharmaceutically acceptable" is employed herein to refer to those compounds, materials, compositions, and/or dosage forms which are, within the scope of sound medical judgment, suitable for use in contact with the tissues of patients without excessive toxicity, irritation, allergic response, or other problem or complication commensurate with a reasonable risk/benefit ratio.

The term "therapeutically effective amount" means the total amount of each active component that is sufficient to show a meaningful patient benefit, e.g., a sustained reduction in viral load. When applied to an individual active ingredient, administered alone, the term refers to that ingredient alone. When applied to a combination, the term refers to combined amounts of the active ingredients that result in the therapeutic effect, whether administered in combination, serially or simultaneously.

The terms "treat" and "treating" refers to: (i) preventing a disease, disorder or condition from occurring in a patient which may be predisposed to the disease, disorder and/or condition but has not yet been diagnosed as having it; (ii) inhibiting the disease, disorder or condition, i.e., arresting its development; and/or (iii) relieving the disease, disorder or condition, i.e., causing regression of the disease, disorder and/or condition.

Where used in naming compounds of the present disclosure, the designations P1', P1, P2, P2*, P3, and P4, as used herein, map the relative positions of the amino acid residues of a protease inhibitor binding relative to the binding of the natural peptide cleavage substrate. Cleavage occurs in the natural substrate between P1 and P1' where the nonprime positions designate amino acids starting from the C-terminus end of the peptide natural cleavage site extending towards the *N*-terminus; whereas, the prime positions emanate from the *N*-terminus end of the cleavage site designation and extend toward the C-terminus. For example, P1' refers to the first position away from the right hand end of the C-terminus of the cleavage site (i.e. *N*-terminus first position); whereas P1 starts the numbering from the left hand side of the C-terminus cleavage site, P2: second position from the C-terminus, etc.). (see Berger A. & Schechter I., Transactions of the Royal Society London series (1970), B257, 249-264].

Asymmetric centers exist in the compounds of the present disclosure. For example, the compounds may include P1 cyclopropyl element of formula

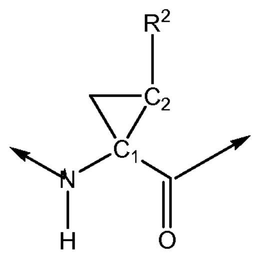

P1

wherein $C_1$ and $C_2$ each represent an asymmetric carbon atom at positions 1 and 2 of the cyclopropyl ring.

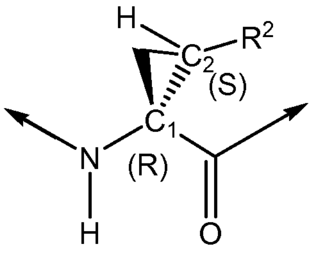

(1R, 2S)
$R^2$ is syn to carbonyl

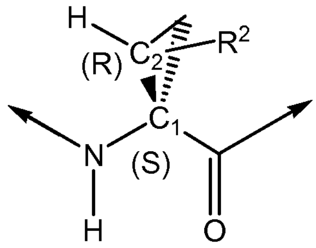

(1S, 2R)
$R^2$ is syn to carbonyl

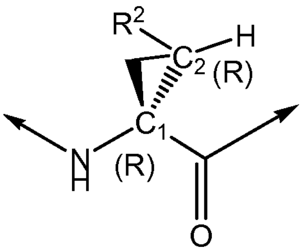

(1R, 2R)
$R^2$ is syn to amide

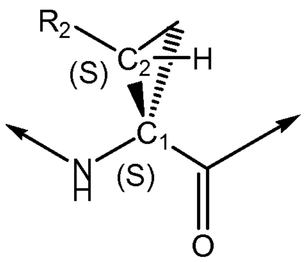

(1S, 2S)
$R^2$ is syn to amide

It should be understood that the disclosure encompasses all stereochemical forms, or mixtures thereof, which possess the ability to inhibit HCV protease.

Certain compounds of the present disclosure may also exist in different stable conformational forms which may be separable. Torsional asymmetry due to restricted rotation about an asymmetric single bond, for example because of steric hindrance or ring strain, may permit separation of different conformers. The present disclosure includes each conformational isomer of these compounds and mixtures thereof.

Certain compounds of the present disclosure may exist in zwitterionic form and the present disclosure includes each zwitterionic form of these compounds and mixtures thereof.

When it is possible that, for use in therapy, therapeutically effective amounts of a compound of formula (I), as well as pharmaceutically acceptable salts thereof, may be administered as the raw chemical, it is possible to present the active ingredient as a pharmaceutical composition. Accordingly, the disclosure further provides pharmaceutical compositions, which include therapeutically effective amounts of compounds of formula (I) or pharmaceutically acceptable salts thereof, and one or more pharmaceutically acceptable carriers, diluents, or excipients. The compounds of formula (I) and pharmaceutically acceptable salts thereof, are as described above. The carrier(s), diluent(s), or excipient(s) must be acceptable in the sense of being compatible with the other ingredients of the formulation and not deleterious to the recipient thereof. In accordance with another aspect of the disclosure there is also provided a process for the preparation of a pharmaceutical formulation including admixing a compound of formula (I), or a pharmaceutically acceptable salt thereof, with one or more pharmaceutically acceptable carriers, diluents, or excipients.

Pharmaceutical formulations may be presented in unit dose forms containing a predetermined amount of active ingredient per unit dose. Dosage levels of between about 0.01 and about 150 milligram per kilogram ("mg/kg") body weight per day, preferably between about 0.05 and about 100 mg/kg body weight per day of the compounds of the disclosure are typical in a monotherapy for the prevention and treatment of HCV mediated disease. Typically, the pharmaceutical compositions of this disclosure will be administered from about 1 to about 5 times per day or alternatively, as a continuous infusion. Such administration can be used as a chronic or acute therapy. The amount of active ingredient that may be combined with the carrier materials to produce a single dosage form will vary depending on the condition being treated, the severity of the condition, the time of administration, the route of administration, the rate of excretion of the compound employed, the duration of treatment, and the age, gender, weight, and condition of the patient. Preferred unit dosage formulations are those containing a daily dose or sub-dose, as herein above recited, or an appropriate fraction thereof, of an active ingredient. Generally, treatment is initiated with small dosages substantially less than the optimum dose of the compound. Thereafter, the dosage is increased by small increments until the optimum effect under

the circumstances is reached. In general, the compound is most desirably administered at a concentration level that will generally afford antivirally effective results without causing any harmful or deleterious side effects.

When the compositions of this disclosure comprise a combination of a compound of the disclosure and one or more additional therapeutic and/or prophylactic agent, both the compound and the additional agent can be present in a dose that is less than or equal to the dosage normally administered in a monotherapy regimen. The compositions of this disclosure may be co-formulated with one or more additional therapeutic or prophylactic agents, for example, in the form of a monolithic and/or bi/multi-layer tablet or may be administered separately from the therapeutic or prophylactic agent(s).

Pharmaceutical formulations may be adapted for administration by any appropriate route, for example by the oral (including buccal or sublingual), rectal, nasal, topical (including buccal, sublingual, or transdermal), vaginal, or parenteral (including subcutaneous, intracutaneous, intramuscular, intra-articular, intrasynovial, intrasternal, intrathecal, intralesional, intravenous, or intradermal injections or infusions) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil emulsions.

For instance, for oral administration in the form of a tablet or capsule, the active drug component can be combined with an oral, non-toxic pharmaceutically acceptable inert carrier such as ethanol, glycerol, water, and the like. Powders are prepared by comminuting the compound to a suitable fine size and mixing with a similarly comminuted pharmaceutical carrier such as an edible carbohydrate, as, for example, starch or mannitol. Flavoring, preservative, dispersing, and coloring agent can also be present.

Capsules are made by preparing a powder mixture, as described above, and filling formed gelatin sheaths. Glidants and lubricants such as colloidal silica, talc, magnesium stearate, calcium stearate, or solid polyethylene glycol can be added to the powder mixture before the filling operation. A disintegrating or solubilizing agent such as agar-agar, calcium carbonate, or sodium carbonate can also be added to improve the availability of the medicament when the capsule is ingested.

Moreover, when desired or necessary, suitable binders, lubricants, disintegrating agents, and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, and the like. Lubricants used in these dosage forms include sodium oleate, sodium chloride, and the like. Disintegrators include, without limitation, starch, methyl cellulose, agar, betonite, xanthan gum, and the like. Tablets are formulated, for example, by preparing a powder mixture, granulating or slugging, adding a lubricant and disintegrant, and pressing into tablets. A powder mixture is prepared by mixing the compound, suitable comminuted, with a diluent or base as described above, and optionally, with a binder such as carboxymethylcellulose, an aliginate, gelating, or polyvinyl pyrrolidone, a solution retardant such as paraffin, a resorption accelerator such as a quaternary salt and/or and absorption agent such as betonite, kaolin, or dicalcium phosphate. The powder mixture can be granulated by wetting with a binder such as syrup, starch paste, acadia mucilage, or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compounds of the present disclosure can also be combined with a free flowing inert carrier and compressed into tablets directly without going through the granulating or slugging steps. A clear or opaque protective coating consisting of a sealing coat of shellac, a coating of sugar or polymeric material, and a polish coating of wax can be provided. Dyestuffs can be added to these coatings to distinguish different unit dosages.

Oral fluids such as solution, syrups, and elixirs can be prepared in dosage unit form so that a given quantity contains a predetermined amount of the compound. Syrups can be prepared by dissolving the compound in a suitably flavored aqueous solution, while elixirs are prepared through the use of a non-toxic vehicle. Solubilizers and emulsifiers such as ethoxylated isostearyl alcohols and polyoxyethylene sorbitol ethers, preservatives, flavor additive such as peppermint oil or natural sweeteners, or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax, or the like.

The compounds of formula (I), and pharmaceutically acceptable salts thereof, can also be administered in the form of liposome delivery systems, such as small unilamellar vesicles, large unilamellar vesicles, and multilamellar vesicles. Liposomes can be formed from a variety of phopholipids, such as cholesterol, stearylamine, or phophatidylcholines.

The compounds of formula (I) and pharmaceutically acceptable salts thereof may also be delivered by the use of monoclonal antibodies as individual carriers to which the compound molecules are coupled. The compounds may

also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyleneoxidepolylysine substituted with palitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates, and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols, or oils.

For treatments of the eye or other external tissues, for example mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in oil base.

Pharmaceutical formulations adapted for topical administrations to the eye include eye drops wherein the active ingredient is dissolved or suspended in a suitable carrier, especially an aqueous solvent.

Pharmaceutical formulations adapted for topical administration in the mouth include lozenges, pastilles, and mouth washes.

Pharmaceutical formulations adapted for rectal administration may be presented as suppositories or as enemas.

Pharmaceutical formulations adapted for nasal administration wherein the carrier is a solid include a course powder which is administered in the manner in which snuff is taken, i.e., by rapid inhalation through the nasal passage from a container of the powder held close up to the nose. Suitable formulations wherein the carrier is a liquid, for administration as a nasal spray or nasal drops, include aqueous or oil solutions of the active ingredient.

Pharmaceutical formulations adapted for administration by inhalation include fine particle dusts or mists, which may be generated by means of various types of metered, dose pressurized aerosols, nebulizers, or insufflators.

Pharmaceutical formulations adapted for vaginal administration may be presented as pessaries, tampons, creams, gels, pastes, foams, or spray formulations.

Pharmaceutical formulations adapted for parenteral administration include aqueous and non-aqueous sterile injection solutions which may contain anti-oxidants, buffers, bacteriostats, and soutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules, and tablets.

It should be understood that in addition to the ingredients particularly mentioned above, the formulations may include other agents conventional in the art having regard to the type of formulation in question, for example those suitable for oral administration may include flavoring agents.

Table 1 below lists some illustrative examples of compounds that can be administered with the compounds of this disclosure. The compounds of the disclosure can be administered with other anti-HCV activity compounds in combination therapy, either jointly or separately, or by combining the compounds into a composition.

*Table 1*

| *Brand Name* | *Physiological Class* | *Type of Inhibitor or Target* | *Source Company* |
|---|---|---|---|
| NIM811 | | Cyclophilin Inhibitor | Novartis |
| Zadaxin | | Immunomodulator | Sciclone |
| Suvus | | Methylene blue | Bioenvision |
| Actilon (CPG10101) | | TLR9 agonist | Coley |
| Batabulin (T67) | Anticancer | β-tubulin inhibitor | Tularik Inc., South San Francisco, CA |

(continued)

| *Brand Name* | *Physiological Class* | *Type of Inhibitor or Target* | *Source Company* |
|---|---|---|---|
| ISIS 14803 | Antiviral | antisense | ISIS Pharmaceutical s Inc, Carlsbad, CA/Elan Phamaceuticals Inc., New York, NY |
| Summetrel | Antiviral | antiviral | Endo Pharmaceutical s Holdings Inc., Chadds Ford, PA |
| GS-9132 (ACH-806) | Antiviral | HCV Inhibitor | Achillion / Gilead |
| Pyrazolopyrimidine compounds and salts From WO-2005047288 26 May 2005 | Antiviral | HCV Inhibitors | Arrow Therapeutics Ltd. |
| Levovirin | Antiviral | IMPDH inhibitor | Ribapharm Inc., Costa Mesa, CA |
| Merimepodib (VX-497) | Antiviral | IMPDH inhibitor | Vertex Pharmaceutical s Inc., Cambridge, MA |
| XTL-6865 (XTL-002) | Antiviral | monoclonal antibody | XTL Biopharmaceuti cals Ltd., Rehovot, Isreal |
| Telaprevir (VX-950, LY-570310) | Antiviral | NS3 serine protease inhibitor | Vertex Pharmaceutical s Inc., Cambridge, MA/ Eli Lilly and Co. Inc., Indianapolis, IN |
| HCV-796 | Antiviral | NS5B Replicase Inhibitor | Wyeth / Viropharma |
| NM-283 | Antiviral | NS5B Replicase Inhibitor | Idenix / Novartis |
| GL-59728 | Antiviral | NS5B Replicase Inhibitor | Gene Labs / Novartis |
| GL-60667 | Antiviral | NS5B Replicase Inhibitor | Gene Labs / Novartis |
| 2'C MeA | Antiviral | NS5B Replicase Inhibitor | Gilead |
| PSI 6130 | Antiviral | NS5B Replicase Inhibitor | Roche |
| R1626 | Antiviral | NS5B Replicase Inhibitor | Roche |
| 2'C Methyl adenosine | Antiviral | NS5B Replicase Inhibitor | Merck |
| JTK-003 | Antiviral | RdRp inhibitor | Japan Tobacco Inc., Tokyo, Japan |
| Levovirin | Antiviral | ribavirin | ICN Pharmaceutical s, Costa Mesa, CA |
| Ribavirin | Antiviral | ribavirin | Schering-Plough Corporation, Kenilworth, NJ |
| Viramidine | Antiviral | Ribavirin Prodrug | Ribapharm Inc., Costa Mesa, CA |
| Heptazyme | Antiviral | ribozyme | Ribozyme Pharmaceutical s Inc., Boulder, CO |

(continued)

| *Brand Name* | *Physiological Class* | *Type of Inhibitor or Target* | *Source Company* |
|---|---|---|---|
| BILN-2061 | Antiviral | serine protease inhibitor | Boehringer Ingelheim Pharma KG, Ingelheim, Germany |
| SCH 503034 | Antiviral | serine protease inhibitor | Schering Plough |
| Zadazim | Immune modulator | Immune modulator | SciClone Pharmaceutical s Inc., San Mateo, CA |
| Ceplene | Immunomodulator | immune modulator | Maxim Pharmaceutical s Inc., San Diego, CA |
| CellCept | Immunosuppressant | HCV IgG immunosuppressant | F. Hoffmann-La Roche LTD, Basel, Switzerland |
| Civacir | Immunosuppressant | HCV IgG immunosuppressant | Nabi Biopharmaceuti cals Inc., Boca Raton, FL |
| Albuferon - α | Interferon | albumin IFN-α2b | Human Genome Sciences Inc., Rockville, MD |
| Infergen A | Interferon | IFN alfacon-1 | InterMune Pharmaceutical s Inc., Brisbane, CA |
| Omega IFN | Interferon | IFN-ω | Intarcia Therapeutics |
| IFN-β and EMZ701 | Interferon | IFN-β and EMZ701 | Transition Therapeutics Inc., Ontario, Canada |
| Rebif | Interferon | IFN-β1a | Serono, Geneva, Switzerland |
| Roferon A | Interferon | IFN-α2a | F. Hoffmann-La Roche LTD, Basel, Switzerland |
| Intron A | Interferon | IFN-α2b | Schering-Plough Corporation, Kenilworth, NJ |
| Intron A and Zadaxin | Interferon | IFN-α2b/α1-thymosin | RegeneRx Biopharmiceuti cals Inc., Bethesda, MD/ SciClone Pharmaceutical s Inc, San Mateo, CA |
| Rebetron | Interferon | IFN-α2b/ribavirin | Schering-Plough Corporation, Kenilworth, NJ |
| Actimmune | Interferon | INF-γ | InterMune Inc., Brisbane, CA |
| Interferon-β | Interferon | Interferon-β-1a | Serono |
| Multiferon | Interferon | Long lasting IFN | Viragen/Valenti s |
| Wellferon | Interferon | lymphoblastoid IFN-αn1 | Glaxo SmithKli ne plc, Uxbridge, UK |
| Omniferon | Interferon | natural IFN-α | Viragen Inc., Plantation, FL |
| Pegasys | Interferon | PEGylated IFN-α2a | F. Hoffmann-La Roche LTD, Basel, Switzerland |
| Pegasys and Ceplene | Interferon | PEGylated IFN-α2a/ immune modulator | Maxim Pharmaceutical s Inc., San Diego, CA |
| Pegasys and Ribavirin | Interferon | PEGylated IFN-α2a/ribavirin | F. Hoffmann-La Roche LTD, Basel, Switzerland |

(continued)

| *Brand Name* | *Physiological Class* | *Type of Inhibitor or Target* | *Source Company* |
|---|---|---|---|
| PEG-Intron | Interferon | PEGylated IFN-$\alpha$2b | Schering-Plough Corporation, Kenilworth, NJ |
| PEG-Intron / Ribavirin | Interferon | PEGylated IFN-$\alpha$2b/ribavirin | Schering-Plough Corporation, Kenilworth, NJ |
| IP-501 | Liver protection | antifibrotic | Indevus Pharmaceuticals Inc., Lexington, MA |
| IDN-6556 | Liver protection | caspase inhibitor | Idun Pharmaceutical s Inc., San Diego, CA |
| ITMN-191 (R-7227) | Antiviral | serine protease inhibitor | InterMune Pharmaceuticals Inc., Brisbane, CA |
| GL-59728 | Antiviral | NS5B Replicase Inhibitor | Genelabs |
| ANA-971 | Antiviral | TLR-7 agonist | Anadys |
| MK 78009 | Antiviral | serine protease inhibitor | Merck |
| TMC-435350 | Antiviral | serine protease inhibitor | Tibotec |

The compounds of the disclosure may also be used as laboratory reagents. Compounds may be instrumental in providing research tools for designing of viral replication assays, validation of animal assay systems and structural biology studies to further enhance knowledge of the HCV disease mechanisms. Further, the compounds of the present disclosure are useful in establishing or determining the binding site of other antiviral compounds, for example, by competitive inhibition.

The compounds of this disclosure may also be used to treat or prevent viral contamination of materials and therefore reduce the risk of viral infection of laboratory or medical personnel or patients who come in contact with such materials, e.g., blood, tissue, surgical instruments and garments, laboratory instruments and garments, and blood collection or transfusion apparatuses and materials.

This disclosure is intended to encompass compounds having formula (I) when prepared by synthetic processes or by metabolic processes including those occurring in the human or animal body (*in vivo*) or processes occurring *in vitro.*

The present disclosure will now be described in connection with certain embodiments. The abbreviations used in the present application, including particularly in the illustrative schemes and examples which follow, are well-known to those skilled in the art. Some of the abbreviations used are as follows: $Et_3N$ for triethylamine; DPPA for diphenylphosphorylazide; h or hr or hrs for hours; EtOAc for ethyl acetate; Ph for phenyl; min or mins for minutes; RT or Rt or rt for room temperature or retention time (context will dictate); DMSO for dimethylsulfoxide; THF for tetrahydrofuran; n-BuLi for n-butyllithium; i-Pr for isopropyl; i-PrO for ispropoxy; MeOH for methanol; TMS for trimethylsilyl, t-Bu for tert-butyl; t-BuO for tert-butoxy; Ts or p-Ts for para-tolylsulfonyl; $iPr_2EtN$ or DIPEA for diisopropylethylamine; HATU for O-(7-azabenzotriazol-1-yl)-N,N,N',N'-tetramethyluronium hexafluorophosphate; Et for ethyl; and $Et_2O$ for diethyl ether.

The starting materials useful to synthesize the compounds of the present disclosure are known to those skilled in the art and can be readily manufactured or are commercially available.

The following methods set forth below are provided for illustrative purposes and are not intended to limit the scope of the claims. It will be recognized that it may be necessary to prepare such a compound in which a functional group is protected using a conventional protecting group then to remove the protecting group to provide a compound of the present disclosure. The details concerning the use of protecting groups in accordance with the present disclosure are known to those skilled in the art.

*Example 1001: Preparation of Compounds 1001*

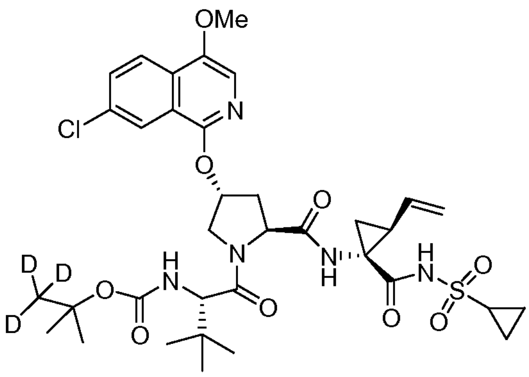

Compound 1001

Scheme 1

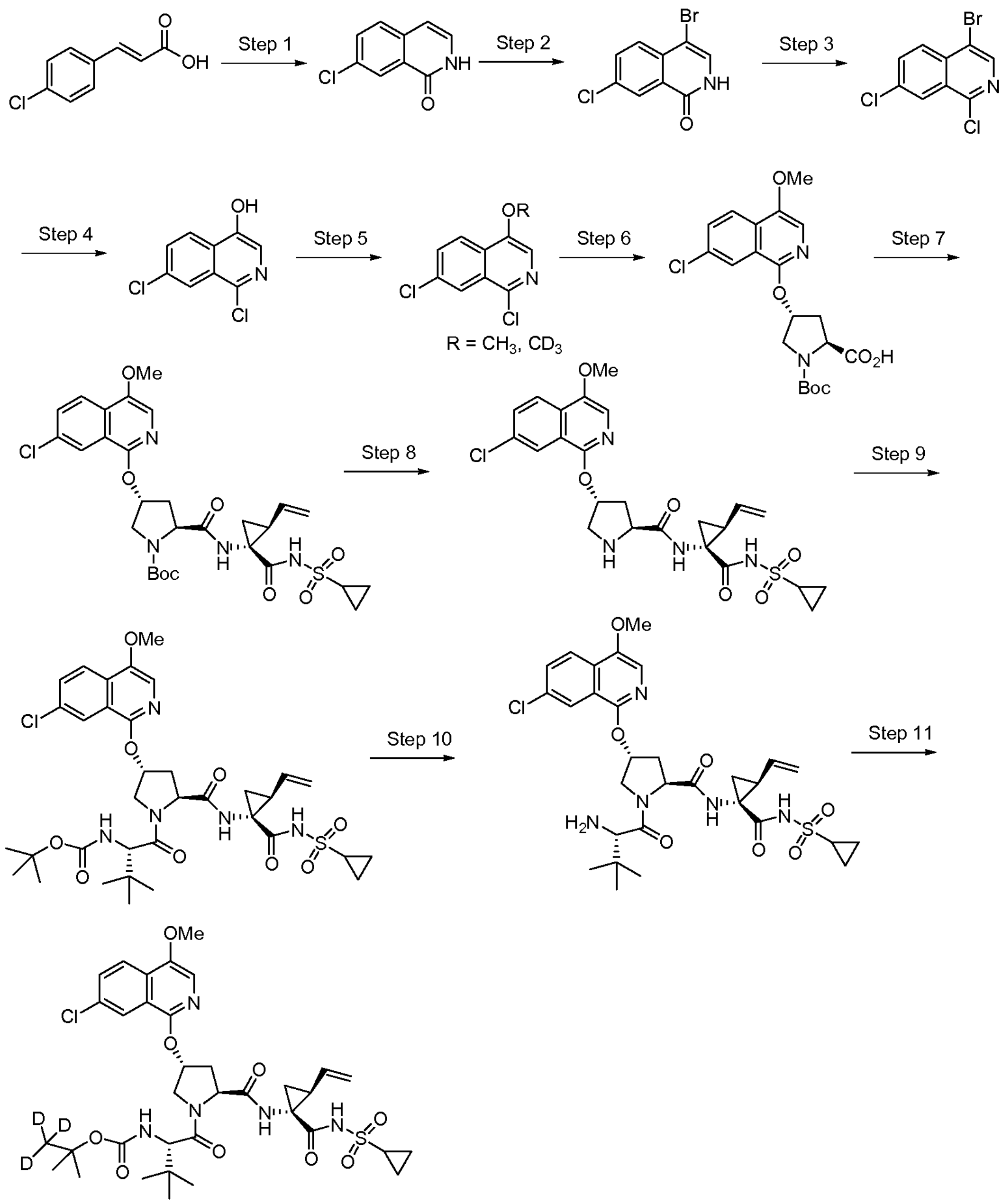


*Step 1*

To a solution of (*E*)-3-(4-chlorophenyl)acrylic acid (18.3 g, 0.1 mol) and $Et_3N$ (20.2 g, 0.2 mol) in benzene (100 mL) was added dropwise DPPA (27.5 g, 0.1 mol). After stirring for 2 h, the solution was concentrated and purified by chromatography (Biotage, mobile phase 20/80 EtOAc/hexanes) to give 16 g of an intermediate azide as a solid. This intermediate was dissolved in 100 mL of $Ph_2CH_2$ and the resulting mixture was slowly heated to 90 °C over a 30 min time period. The reaction mixture was heated to reflux and maintained at this temperature for 3h. After cooling to RT, a solid precipitated which was collected by filtration and washed with toluene to provide 9.5 g of 7-chloroisoquinolin-1(2H)-one (53%). $^1H$ NMR (400 MHz, $CD_3OD$) δ ppm 6.66 (d, *J*=7.05 Hz, 1 H), 7.18 (d, *J*=7.05 Hz, 1 H), 7.66 (s, 1 H) 7.67 (d, *J*=2.01 Hz, 1 H), 8.24 (d, J=2.27 Hz, 1 H); $^{13}C$ NMR (101 MHz, DMSO-$D_6$) δ ppm 104.05, 125.62, 127.21, 128.54, 129.52, 130.77, 132.43, 136.55, 160.72; LC/MS, MS m/z $(M+H)^+$ 180.

*Step 2*

A slurry of the product of Step 1, Example 1001, 7-chloroisoquinolin-1(2*H*)-one (36.33 g, 203 mmol) and N-bromosuccinimide (39.74 g, 223.3 mmol) in anhydrous $CH_3CN$ (500 mL) was slowly heated to a gentle reflux over a period of approximately 2 h and maintained at a gentle reflux for 1.5 h. The reaction was monitored by LC/MS and, when complete, the slurry, was slowly cooled to room temperature over a period of 3 h. The precipitated solid was collected by filtration and washed with $CH_3CN$ (100 mL x 3) to provide 47 g (90%) of 4-bromo-7-chloroisoquinolin-1(2H)-one. This material was used in the next step without further purification. $^1H$ NMR (400 MHz, $CD_3OD$) $\delta$ ppm 7.46(s, 1H), 7.81 (dd, *J*=8.40, 2.00 Hz, 1 H), 7.88 (d, *J*=8.8 Hz, 1 H), 8.27(d, *J*=2.00 Hz, 1 H); $^{13}C$ NMR (101 MHz, DMSO-$D_6$) $\delta$ ppm 96.68, 126.34, 127.58, 127.71, 130.73, 132.20, 133.47, 134.46, 159.88; LC/MS, MS m/z $(M+H)^+$ 258.

*Step 3*

A heterogeneous solution of the product of Step 2, Example 1001, 4-bromo-7-chloroisoquinolin-1(2H)-one (47 g, 182 mmol) in $POCl_3$ (200 mL, 2.15 mol) was slowly heated to reflux over a period of 1 h. It should be noted that during this heating process, the reaction mixture became homogeneous. The reaction mixture was maintained at reflux for 4 h before being cooled to room temperature and concentrated in vacuo to remove excess $POCl_3$. To ensure complete removal of residual $POCl_3$, the residue was dissolved in $CH_2Cl_2$ or, alternatively, toluene and concentrated in vacuo. This process was repeated as necessary. (Note that $POCl_3$ was properly disposed of in glass bottles which were labeled accordingly). The residue was taken-up into 600 mL of $CH_2Cl_2$, cooled to -35 °C, neutralized and subsequently basified carefully with 1N NaOH (400 mL) until the mixture was slightly basic (pH = 8). The organic layer was separated, washed with $H_2O$, dried over $MgSO_4$ and concentrated in vacuo. The residual solid was crystallized from EtOAc (approximately 50 mL) to give 32 g of 4-bromo-1,7-dichloroisoquinoline. The mother liquor from the crystallization process was concentrated and purified by Biotage (16% EtOAc in hexanes) to provide an additional 4 g of 4-bromo-1,7-dichloroisoquinoline as a solid. In total 36 g (73 %) of 4-bromo-1,7-dichloroisoquinoline was obtained. $^1H$ NMR (400 MHz, $CDCl_3$) $\delta$ ppm 7.80 (dd, *J*=8.81, 2.01 Hz, 1 H), 8.14 (d, *J*=9.06 Hz, 1 H), 8.34 (d, *J*=1.76 Hz, 1 H), 8.48 (s, 1 H); $^{13}C$ NMR (101 MHz, DMSO-$D_6$) $\delta$ ppm 118.39, 125.06, 127.59, 128.71, 133.89, 134.14, 134.93, 143.18, 148.98; LC/MS, MS m/z $(M+H)^+$ 275.

*Step 4*

To a slurry of solution of the product of Step 3, Example 1001, 4-bromo-1,7-dichloroisoquinoline (22.16 g, 80 mmol) in THF (500 ml) maintained at -78 °C was added 1.6 M n-BuLi in hexanes (100 mL, 160 mmol) dropwise *via* cannula over 15 min (the internal temperature was maintained <-65 °C). The solution was stirred for 0.5 h, and $(i\text{-PrO})_3B$ (37 ml, 160 mmol) added dropwise *via* syringe over 10 min (the internal temperature was maintained <-65 °C). The reaction mixture was stirred for 0.5 h and 30% $H_2O_2$ (80 ml, 776 mmol) added dropwise *via* an addition funnel over 10 min (the internal temperature rose to -60 °C during this addition), followed by addition of 1N NaOH solution (80 ml, 80 mmol). The cooling bath was removed and the reaction mixture allowed to warm to room temperature and stirred for an additional 1 h. After confirming the completion of the reaction by LC/MS, the reaction mixture was cooled to -40 °C, and a solution of 100 g of $Na_2SO_3$ (0.793 moles) in 400 mL of $H_2O$ added dropwise over 30 min (the internal temperature was maintained between 5-10°C). The resulting slurry was neutralized with 6 N HCl (approximately 50 ml) at 0 °C to provide a pH ~ 6. The mixture was diluted with 500 ml of EtOAc and decanted into a 2 L separatory funnel. To the remaining solid in the reaction vessel was added 500 mL of $H_2O$ and 300 ml of EtOAc and the mixture neutralized with 6 N HCl (approximately 20 ml). The organic layers were combined in a separatory funnel, washed with brine (300 ml x 3) and $H_2O$ (200 ml x 3). The organic phase was dried over $MgSO_4$, filtered to remove the drying agent, and concentrated to give a crude product which was triturated with 50 ml of EtOAc. The resulting solid was collected by filtration, rinsed with EtOAc (3 x 25 ml) and dried to provide 1,7-dichloroisoquinolin-4-ol (2 runs: 12.0 g, 70% and 13.8 g, 81%). The filtrates were combined, concentrated and purified by Biotage (35% EtOAc in hexanes) to give an additional 2.1 g of BMS-796007. In total, 27.9 g (82%) of 1,7-dichloroisoquinolin-4-ol was obtained. $^1H$ NMR (400 MHz, $CD_3OD$) $\delta$ ppm 4.05 (s, 3 H), 7.4 (s, 1 H), 7.76 (dd, *J*=8.8, 2, Hz, 1 H), 8.16 (d, *J*=2 Hz, 1 H), 8.23 (d, *J*=8.8 Hz, 1 H); $^{13}C$ NMR (101 MHz, DMSO-$D_6$) $\delta$ ppm 123.78, 124.66, 125.62, 127.03, 127.71, 130.72, 133.80, 137.63; 148.88; LC/MS, MS (m/z) $(M+H)^+$ 213.

*Step 5*

*Preparation of 1,7-dichloro-4-methoxyisoquinoline:*

To a slurry of the product of Step 4, Example 1001, 1,7-dichloroisoquinolin-4-ol (16 g, 75.5 mmol) in MeOH/$CH_3CN$ (30 mL/300 mL) maintained at 0 °C was added dropwise a 2 M solution of $TMSCHN_2$ in hexanes (60 ml,

120 mmol). The reaction mixture was warmed to room temperature and stirred for 14 h. The solution was concentrated and the residual solid recrystallized from EtOAc (about 50 mL) to give 8.1 g of 1,7-dichloro-4-methoxyisoquinoline which was washed with 25% EtOAc in hexanes. The mother liquid was concentrated and purified by Biotage (16 % EtOAc in hexanes) to provide an additional 3.2 g of 1,7-dichloro-4-methoxyisoquinoline as a solid. In total, 11.3 g (66%) of 1,7-dichloro-4-methoxyisoquinoline was obtained. $^1$H NMR (400 MHz, $CDCl_3$) δ ppm 4.05 (s, 3 H), 7.67 (dd, *J*=9.06, 2.01 Hz, 1 H), 7.80 (s, 1 H), 8.16 (d, *J*=8.81 Hz, 1 H), 8.23 (d, J=2.01 Hz, 1 H); $^{13}$C NMR (101 MHz, DMSO-$D_6$) δ ppm 56.68, 122.70, 123.99, 124.14, 126.67, 127.83, 131.43, 134.10, 139.75, 149.94; LC/MS, MS m/z $(M+H)^+$ 228.

*Preparation of 1,7-dichloro-4-($D_3$-methoxy)isoquinoline:*

A mixture of Step 4, Example 1001, 1,7-dichloroisoquinolin-4-ol (321 mg, 1.5 mmol), $ICD_3$ (217 mg, 1.500 mmol), and $K_2CO_3$ (622 mg, 4.50 mmol) in acetone (10 mL) was refluxed for 20 h. After filtration, the filtrate was concentrated and purified by Biotage eluting with 10% ethyl acetate in hexane to give 250 mg of the desired product as a solid. LC/MS, MS (m/z) $(M+H)^+$ 231.09.

*Step 6*

To a mixture of the product of Step 5, Example 1001, 1,7-dichloro-4-methoxyisoquinoline (4.52 g, 20 mmol), (2*S*,4*R*)-1-(tert-butoxycarbonyl)-4-hydroxypyrrolidine-2-carboxylic acid (5.08 g, 22 mmol) and t-BuOK (6.72 g, 60 mmol) was added DMSO (200 mL) with stirring at 10°C. The resulting slurry was sonicated for 30 min to provide a homogeneous solution which was stirred at room temperature for 3 h. The reaction mixture was cooled to 0 °C and quenched by the addition of $H_2O$ (50 ml). The mixture was neutralized, then acidified, to a final pH of 5 by the careful addition of 1 N aqueous HCl. The mixture was extracted with EtOAc (400 mL), and the organic layer washed with brine (200 mL), $H_2O$ (200 mLx2) before being dried over $MgSO_4$ and *concentrated in vacuo* to provide 8.36 g of crude solid (2S,4R)-1-(tert-butoxycarbonyl)-4-(7-chloro-4-methoxyisoquinolin-1-yloxy)pyrrolidine-2-carboxylic acid. This material was used in the next step without further purification. $^1$H NMR (400 MHz, $CD_3OD$) δ ppm 2.34 - 2.47 (m, 1 H), 2.62-2.77 (m, 1 H), 3.70 - 3.92 (m, 2 H), 4.42 - 4.59 (m, 1 H), 5.65 (brs, 1 H), 7.54 (s, 1 H), 7.68 (dd, *J*=8.81, 2.01 Hz, 1 H), 8.02 - 8.13 (m, 2 H); $^{13}$C NMR (126 MHz, DMSO-$D_6$) δ ppm 13.90, 14.04, 20.71, 22.02, 27.84, 27.98, 30.91, 35.00, 35.87, 51.84, 52.08, 56.21, 57.49, 57.80, 59.70, 73.32, 73.87, 79.14, 79.19, 119.11, 119.77, 122.38, 123.35, 128.50, 130.95, 132.25, 145.70, 151.94, 153.25, 153.71, 173.51, 173.98; MS $(M+H)^+$ 423.

*Step 7*

To a solution of the product of Step 6, Example 1001, (2*S*,4*R*)-1-(tert-butoxycarbonyl)-4-(7-chloro-4-methoxyisoquinolin-1-yloxy)pyrrolidine-2-carboxylic acid (8.36 g, 19.8 mmol), (1*R*,2*S*)-1-amino-N-(cyclopropylsulfonyl)-2-vinylcyclopropanecarboxamide TsOH salt (9.64g, 24 mmol), and $iPr_2EtN$ (17.4 mL, 100 mmol) in $CH_2Cl_2$ (200 mL) was added HATU (11.4 g, 31 mmol). The reaction mixture was stirred for 16 h, *concentrated in vacuo* and the residue dissolved in EtOAc (300 mL) and washed sequentially with 1 N HCl (50 mL x 3), water (30 mL x 2), and brine (50 ml x 2). The organics were dried over $MgSO_4$, concentrated, and purified using a Biotage (25% acetone in hexanes) to provide 11.5 g of crude product (2*S*,4*R*)-tert-butyl 4-(7-chloro-4-methoxyisoquinolin-1-yloxy)-2-((1*R*,2*S*)-1-(cyclopropylsulfonylcarbamoyl)-2-vinylcyclopropylcarbamoyl)pyrrolidine-1-carboxylate. This compound was purified by crystallizing from MeOH (40 mL) to afford 11 g (88%) of a crystalline solid. $^1$H NMR (400 MHz, $CD_3OD$) δ ppm 1.03-1.31 (m, 8 H), 1.43 (s, 9H), 1.88 (dd, *J*=8.06, 5.54 Hz, 1 H), 2.17 - 2.36 (m, 2 H), 2.53 (dd, *J*=13.72, 6.42 Hz, 1 H), 2.90 - 3.03 (m, 1 H), 3.72 - 3.93 (m, 2 H), 4.40 (dd, *J*=9.69, 6.92 Hz, 1 H), 5.13 (d, *J*=10.32 Hz, 1 H), 5.31 (d, *J*=17.12 Hz, 1 H), 5.65-5.93 (m, 2 H), 7.55 (s, 1 H), 7.70 (dd, *J*=8.94, 2.14 Hz, 1 H), 8.06 (d, *J*=2.01 Hz, 1 H), 8.09 (d, *J*=8.81 Hz, 1 H); $^{13}$C NMR (126 MHz, DMSO-$D_6$) δ ppm 5.47, 5.57, 5.75, 19.85, 22.38, 27.90, 27.99, 30.65, 30.72, 32.11, 33.81, 35.11, 36.30, 40.86, 41.59, 48.56, 52.39, 52.76, 56.24, 58.76, 59.21, 73.57, 74.06, 79.28, 80.06, 117.80, 119.16, 119.81, 119.88, 122.14, 123.48, 128.52, 131.00, 132.28, 133.38, 145.72, 151.77, 151.86, 154.06, 168.38, 169.13, 172.46, 173.27; MS: $(M+H)^+$ 635.

*Step 8*

A slurry of the product of Step 7, Example 1001, (2*S*,4*R*)-tert-butyl 4-(7-chloro-4-methoxyisoquinolin-1-yloxy)-2-((1*R*,2*S*)-1-(cyclopropylsulfonylcarbamoyl)-2-vinylcyclopropylcarbamoyl)pyrrolidine-1-carboxylate (6.34 g, 10 mmol) in 50 mL of MeOH containing 3 ml of concentrated HCl was refluxed for 2 h. The solution was cooled to room temperature and *concentrated in vacuo.* The solid residue was taken up in dry $Et_2O$ (50 ml) and the solution *concentrated in vacuo.* This process, repeated five times to ensure complete removal of water and solubilized HCl, provided 6.07 g of (2S,4R)-4-(7-chloro-4-methoxyisoquinolin-1-yloxy)-N-((1R,2S)-1-(cyclopropylsulfonylcarbamoyl)-2-vinylcyclopropyl)pyrrolidine-2-carboxamide as HCl salt (100%). $^1$H NMR (400 MHz, $CD_3OD$) δ ppm 0.96 - 1.21 (m, 3 H), 1.22 - 1.30 (m, 1 H), 1.38

(dd, *J*=9.57, 5.54 Hz, 1 H), 1.95 (dd, *J*=8.06, 5.79 Hz, 1 H), 2.25 - 2.46 (m, 2 H), 2.83 - 3.08 (m, 2 H), 3.75 - 3.90 (m, 2 H), 4.01 (s, 3 H), 4.70 (dd, *J*=10.32, 7.81 Hz, 1 H), 5.10 - 5.20 (m, 1 H), 5.33 (d, *J*=17.12 Hz, 1 H), 5.58 - 5.76 (m, 1 H), 5.88 (s, 1 H), 7.57 (s, 1 H), 7.74 (dd, *J*=8.81, 2.01 Hz, 1 H), 8.12 (d, *J*=9.06 Hz, 1 H), 8.28 (d, *J*=2.01 Hz, 1 H); $^{13}C$ NMR (101 MHz, $CD_3OD$) δ ppm 6.52, 6.65, 22.60, 31.99, 34.63, 37.04, 43.18, 52.95, 56.85, 60.56, 76.08, 119.06, 119.10, 121.65, 123.93, 124.63, 130.72, 132.37, 133.78, 134.76, 148.49, 153.02, 170.08, 170.67; LC/MS MS m/z $(M+H)^+$ 535.

*Step 9*

To a solution of the product of Step 8, Example 1001, (2S,4R)-4-(7-chloro-4-methoxyisoquinolin-1-yloxy)-N-((1R,2S)-1-(cyclopropylsulfonylcarbamoyl)-2-vinylcyclopropyl)pyrrolidine-2-carboxamide as HCl salt (6.07 g, 10 mmol) in 100 mL of $CH_2Cl_2$, maintained at 0°C, was added 8.7 mL of $iPr_2EtN$ (50 mmol) followed by Boc-L-*tert*-leucine (2.772 g, 12 mmol) and HATU (5.7 g, 15 mmol). The reaction mixture was warmed to RT and stirred for 16h before being *concentrated in vacuo* and the residue dissolved in EtOAc (300 mL). The EtOAc solution was washed sequentially with 1N HCl (50 mL x 3), $H_2O$ (30 mL x 2), and brine (50 ml x 2). The organic phase was dried over $MgSO_4$ and concentrated *in vacuo* and the crude product obtained after purification using a Biotage (33% acetone in hexanes) to provide 7 g (94%) of the desired product. $^1H$ NMR (400 MHz, $CD_3OD$) δ ppm 1.00-1.06 (m, 11 H), 1.16 (s, 9 H), 1.14-1.24 (m, 2 H), 1.44 (dd, *J*=9.32, 5.29 Hz, 1 H), 1.88 (dd, *J*=8.06, 5.54 Hz, 1 H), 2.17 - 2.39 (m, 2 H), 2.59 (dd, *J*=13.85, 6.80 Hz, 1 H), 2.87 - 3.02 (m, 1 H), 4.00 (s, 3 H), 4.01 - 4.14 (m, 1 H), 4.17 - 4.24 (m, 1 H), 4.43 (d, *J*=12.09 Hz, 1 H), 4.52 - 4.65 (m, 1 H), 5.12 (d, *J*=10.07 Hz, 1 H), 5.30 (d, *J*=16.87 Hz, 1 H), 5.65 - 5.91 (m, 2 H), 7.56 (s, 1H), 7.68 (d, *J*=9.06 Hz, 1 H), 8.05 (s, 1 H), 8.09 (d, *J*=9.06 Hz, 1 H);MS: $(M+H)^+$ 748.

*Step 10*

To the product of the product of Step 9, Example 1001, tert-butyl (S)-1-((2S,4R)-4-(7-chloro-4-methoxyisoquinolin-1-yloxy)-2-((1R,2S)-1-(cyclopropylsulfonylcarbamoyl)-2-vinylcyclopropylcarbamoyl)pyrrolidin-1-yl)-3,3-dimethyl-1-oxobutan-2-ylcarbamate (2.469 g, 3.3 mmol) was added 4M HCl (8.25 mL, 33.0 mmol) in 1,4-dioxane. The formed solution was stirred at 25 °C for 3 h. After concentration under vacuo, to the residue was added ether (20mL), then concentrated again, repeated the procedure 3 times. House vacuum drying gave 2.36 g (100%) of the crude product as a solid, which was used in the next step without further purification. MS: $(M+H)^+$ 648.50.

*Step 11*

A solution of the product of Step 10, Example 1001, (2S,4R)-1-((S)-2-amino-3,3-dimethylbutanoyl)-4-(7-chloro-4-methoxyisoquinolin-1-yloxy)-N-((1R,2S)-1-(cyclopropylsulfonylcarbamoyl)-2-vinylcyclopropyl)pyrrolidine-2-carboxamide, HCl (30 mg, 0.044 mmol), Reactant 2 (9.56 mg, 0.048 mmol), and N-ethyl-N-isopropylpropan-2-amine (0.038 mL, 0.219 mmol) in $CH_2Cl_2$ (Volume: 3 mL) was stirred for 16 h. After concentration, the residue was purified by prep HPLC to give 21 mg (64%) of the desired product Compound 1001 as a solid. $^1H$ NMR (400 MHz, *MeOD*) δ ppm 0.99 - 1.13 (m, 11 H), 1.17 (s, 6 H), 1.21 - 1.32 (m, 2 H), 1.45 (dd, *J*=9.4, 5.4 Hz, 1 H), 1.89 (dd, *J*=8.2, 5.4 Hz, 1 H), 2.20 - 2.35 (m, 2 H), 2.61 (dd, *J*=13.7, 6.9 Hz, 1 H), 2.91 - 3.01 (m, 1 H), 4.01 (s, 3 H), 4.03 - 4.12 (m, 1 H), 4.18-4.23 (m, 1 H), 4.43 (s, 1 H), 4.57 (dd, *J*=10.2, 7.2 Hz, 1 H), 5.14 (dd, *J*=10.3, 1.5 Hz, 1 H), 5.32 (d, *J*=17.1 Hz, 1 H), 5.71 - 5.85 (m, 2 H), 7.58 (s, 1 H), 7.69 (dd, *J*=8.5, 1.8 Hz, 1 H), 8.06 (d, *J*=1.8 Hz, 1 H), 8.10 (d, *J*=8.8 Hz, 1 H); MS: $(M+H)^+$ 751.31.

*Example 1002: Preparation of Compound 1002*

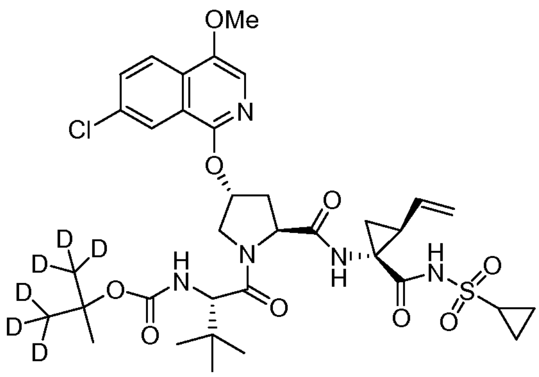

Compound 1002

Compound 1002 was prepared by a similar procedure as that described for the preparation of Compound 1001, MS: $(M+H)^+$ 754.35.

*Example 1003: Preparation of Compound 1003*

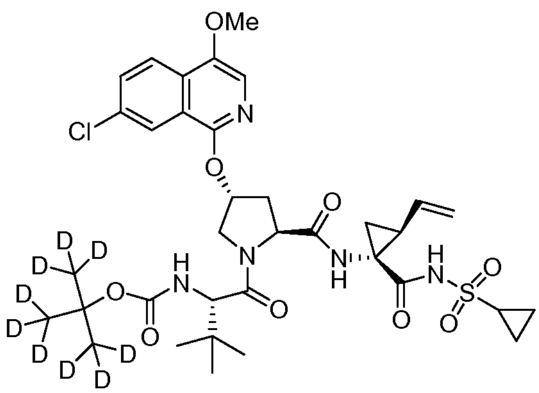

Compound 1003

Compound 1003 was prepared by a similar procedure as that described for the preparation of Compound 1001, MS: $(M+H)^+$ 757.35.

*Biological Studies*

HCV NS3/4A protease complex enzyme assays and cell-based HCV replicon assays can be prepared, conducted and validated using the information known in the art, as follows:

*Generation of recombinant HCV NS3/4A protease complex*

HCV NS3 protease complexes, derived from the BMS strain, H77 strain or J4L6S strain, were generated, as described below. These purified recombinant proteins were generated for use in a homogeneous assay (see below) to provide an indication of how effective compounds of the present disclosure would be in inhibiting HCV NS3 proteolytic activity.

Serum from an HCV-infected patient was obtained from Dr. T. Wright, San Francisco Hospital. An engineered full-length cDNA (compliment deoxyribonucleic acid) template of the HCV genome (BMS strain) was constructed from DNA fragments obtained by reverse transcription-PCR (RT-PCR) of serum RNA (ribonucleic acid) and using primers selected on the basis of homology between other genotype 1a strains. From the determination of the entire genome sequence, a genotype 1a was assigned to the HCV isolate according to the classification of Simmonds et al. (See P

Simmonds, KA Rose, S Graham, SW Chan, F McOmish, BC Dow, EA Follett, PL Yap and H Marsden, J. Clin. Microbiol., 31(6), 1493-1503 (1993)). The amino acid sequence of the nonstructural region, NS2-5B, was shown to be >97% identical to HCV genotype 1a (H77) and 87% identical to genotype 1b (J4L6S). The infectious clones, H77 (1a genotype) and J4L6S (1b genotype) were obtained from R. Purcell (NIH) and the sequences are published in Genbank (AAB67036, see Yanagi,M., Purcell,R.H., Emerson,S.U. and Bukh, J. Proc. Natl. Acad. Sci. U.S.A. 94(16), 8738-8743 (1997); AF054247, see Yanagi,M., St Claire,M., Shapiro,M., Emerson,S.U., Purcell,R.H. and Bukh, J., Virology 244 (1), 161-172. (1998)).

The H77 and J4L6S strains were used for production of recombinant NS3/4A protease complexes. DNA encoding the recombinant HCV NS3/4A protease complex (amino acids 1027 to 1711) for these strains was manipulated as described by P. Gallinari et al. (see Gallinari P, Paolini C, Brennan D, Nardi C, Steinkuhler C, De Francesco R. Biochemistry. 38(17):5620-32, (1999)). Briefly, a three-lysine solubilizing tail was added at the 3'-end of the NS4A coding region. The cysteine in the P1 position of the NS4A-NS4B cleavage site (amino acid 1711) was changed to a glycine to avoid the proteolytic cleavage of the lysine tag. Furthermore, a cysteine to serine mutation was introduced by PCR at amino acid position 1454 to prevent the autolytic cleavage in the NS3 helicase domain. The variant DNA fragment was cloned in the pET21b bacterial expression vector (Novagen) and the NS3/4A complex was expressed in Escherichia. coli strain BL21 (DE3) (Invitrogen) following the protocol described by P. Gallinari et al. (see Gallinari P, Brennan D, Nardi C, Brunetti M, Tomei L, Steinkuhler C, De Francesco R., J Virol. 72(8):6758-69 (1998)) with modifications. Briefly, the NS3/4A protease complex expression was induced with 0.5 millimolar (mM) Isopropyl β-D-1-thiogalactopyranoside (IPTG) for 22 hours (h) at 20°C. A typical fermentation (1 Liter (L)) yielded approximately 10 grams (g) of wet cell paste. The cells were resuspended in lysis buffer (10 mL/g) consisting of 25 mM *N*-(2-Hydroxyethyl)Piperazine-*N*'-(2-Ethane Sulfonic acid) (HEPES), pH 7.5, 20% glycerol, 500 mM Sodium Chloride (NaCl), 0.5% Triton X-100, 1 microgram/milliliter ("μg/mL") lysozyme, 5 mM Magnesium Chloride ($MgCl_2$), 1 μg/ml DnaseI, 5 mM β-Mercaptoethanol (βME), Protease inhibitor-Ethylenediamine Tetraacetic acid (EDTA) free (Roche), homogenized and incubated for 20 minutes (min) at 4°C. The homogenate was sonicated and clarified by ultra-centrifugation at 235000 g for 1 hour at 4 °C. Imidazole was added to the supernatant to a final concentration of 15 mM and the pH adjusted to 8.0. The crude protein extract was loaded on a Nickel-Nitrilotriacetic acid (Ni-NTA) column pre-equilibrated with buffer B (25 mM HEPES, pH 8.0, 20% glycerol, 500 mM NaCl, 0.5% Triton X-100, 15 mM imidazole, 5 mM βME). The sample was loaded at a flow rate of 1 mL/min. The column was washed with 15 column volumes of buffer C (same as buffer B except with 0.2% Triton X-100). The protein was eluted with 5 column volumes of buffer D (same as buffer C except with 200 mM Imidazole).

NS3/4A protease complex-containing fractions were pooled and loaded on a desalting column Superdex-S200 pre-equilibrated with buffer D (25 mM HEPES, pH 7.5, 20% glycerol, 300 mM NaCl, 0.2% Triton X-100, 10 mM βME). Sample was loaded at a flow rate of 1 mL/min. NS3/4A protease complex-containing fractions were pooled and concentrated to approximately 0.5 mg/ml. The purity of the NS3/4A protease complexes, derived from the BMS, H77 and J4L6S strains, were judged to be greater than 90% by SDS-PAGE and mass spectrometry analyses. The enzyme was stored at -80 °C, thawed on ice and diluted prior to use in assay buffer.

*FRET peptide assay to monitor HCV NS3/4A proteolytic activty*

The purpose of this in vitro assay was to measure the inhibition of HCV NS3 protease complexes, derived from the BMS strain, H77 strain or J4L6S strain, as described above, by compounds of the present disclosure. This assay provides an indication of how effective compounds of the present disclosure would be in inhibiting HCV NS3 proteolytic activity.

In order to monitor HCV NS3/4A protease activity, an NS3/4A peptide substrate was used. The substrate was RET S1 (Resonance Energy Transfer Depsipeptide Substrate; AnaSpec, Inc. cat # 22991)(FRET peptide), described by Taliani et al. in Anal. Biochem. 240(2):60-67 (1996). The sequence of this peptide is loosely based on the NS4A/NS4B natural cleavage site for the HCV NS3 protease except there is an ester linkage rather than an amide bond at the cleavage site. The peptide also contains a fluorescence donor, EDANS, near one end of the peptide and an acceptor, DABCYL, near the other end. The fluorescence of the peptide is quenched by intermolecular resonance energy transfer (RET) between the donor and the acceptor, but as the NS3 protease cleaves the peptide the products are released from RET quenching and the fluorescence of the donor becomes apparent.

The peptide substrate was incubated with one of the three recombinant NS3/4A protease complexes, in the absence or presence of a compound of the present disclosure. The inhibitory effects of a compound were determined by monitoring the formation of fluorescent reaction product in real time using a Cytofluor Series 4000.

The reagents were as follow: HEPES and Glycerol (Ultrapure) were obtained from GIBCO-BRL. Dimethyl Sulfoxide (DMSO) was obtained from Sigma. β-Mercaptoethanol was obtained from Bio Rad.

Assay buffer: 50 mM HEPES, pH 7.5; 0.15 M NaCl; 0.1% Triton; 15% Glycerol; 10 mM βME. Substrate: 2 μM final concentration (from a 2 mM stock solution in DMSO stored at -20°C). HCV NS3/4A protease type 1a (1b), 2-3 nM final concentration (from a 5 μM stock solution in 25 mM HEPES, pH 7.5, 20% glycerol, 300 mM NaCl, 0.2% Triton-X100, 10 mM βME). For compounds with potencies approaching the assay limit, the assay was made more sensitive by adding 50 μg/ml Bovine Serum Albumin (Sigma) to the assay buffer and reducing the end protease concentration to 300 pM.

The assay was performed in a 96-well polystyrene black plate from Falcon. Each well contained 25 μl NS3/4A protease complex in assay buffer, 50 μl of a compound of the present disclosure in 10% DMSO/assay buffer and 25 μl substrate in assay buffer. A control (no compound) was also prepared on the same assay plate. The enzyme complex was mixed with compound or control solution for 1 min before initiating the enzymatic reaction by the addition of substrate. The assay plate was read immediately using the Cytofluor Series 4000 (Perspective Biosystems). The instrument was set to read an emission of 340 nm and excitation of 490 nm at 25°C. Reactions were generally followed for approximately 15 min.

The percent inhibition was calculated with the following equation:

$$100-[(\delta F_{inh}/\delta F_{con})x100]$$

where δF is the change in fluorescence over the linear range of the curve. A non-linear curve fit was applied to the inhibition-concentration data, and the 50% effective concentration ($IC_{50}$) was calculated by the use of Excel XLfit software using the equation, y=A+((B-A)/(1+((C/x)^D))).

Compounds of the present disclosure, which were tested against more than one type of NS3/4A complex, were found to have similar inhibitory properties though the compounds uniformly demonstrated greater potency against the 1b strains as compared to the 1a strains.

*Specificity Assays*

The specificity assays were performed to demonstrate the in vitro selectivity of the compounds of the present disclosure in inhibiting HCV NS3/4A protease complex as compared to other serine or cysteine proteases.

The specificities of compounds of the present disclosure were determined against a variety of serine proteases: human neutrophil elastase (HNE), porcine pancreatic elastase (PPE) and human pancreatic chymotrypsin and one cysteine protease: human liver cathepsin B. In all cases a 96-well plate format protocol using a fluorometric Amino-Methyl-Coumarin (AMC) substrate specific for each enzyme was used as described previously (PCT Patent Application No. WO 00/09543) with some modifications to the serine protease assays. All enzymes were purchased from Sigma, EMDbiosciences while the substrates were from Bachem, Sigma and EMDbiosciences.

Compound concentrations varied from 100 to 0.4 μM depending on their potency. The enzyme assays were each initiated by addition of substrate to enzyme-inhibitor pre-incubated for 10 min at room temperature and hydrolysis to 15% conversion as measured on cytofluor.

The final conditions for each assay were as follows:

50 mM Tris(hydroxymethyl) aminomethane hydrochloride (Tris-HCl) pH 8, 0.5 M Sodium Sulfate ($Na_2SO_4$), 50 mM NaCl, 0.1 mM EDTA, 3% DMSO, 0.01% Tween-20 with 5 μM LLVY-AMC and 1 nM Chymotrypsin.

50 M Tris-HCl, pH 8.0, 50 mM NaCl, 0.1mM EDTA, 3% DMSO, 0.02% Tween-20, 5 μM succ-AAPV-AMC and 20 nM HNE or 8 nM PPE;

100 mM NaOAC (Sodium Acetate) pH 5.5, 3% DMSO, 1 mM TCEP (Tris(2-carboxyethyl)phosphine hydrochloride), 5 nM Cathepsin B (enzyme stock activated in buffer containing 20 mM TCEP before use), and 2 μM Z-FR-AMC diluted in $H_2O$. The percentage of inhibition was calculated using the formula:

$$[1-((UV_{inh}-UV_{blank})/(UV_{ctl}-UV_{blank}))] \text{ x } 100$$

A non-linear curve fit was applied to the inhibition-concentration data, and the 50% effective concentration ($IC_{50}$) was calculated by the use of Excel XLfit software.

*Generation of HCV Replicon*

An HCV replicon whole cell system was established as described by Lohmann V, Korner F, Koch J, Herian U, Theilmann L, Bartenschlager R., Science 285(5424):110-3 (1999). This system enabled us to evaluate the effects of

our HCV Protease compounds on HCV RNA replication. Briefly, using the HCV strain 1b sequence described in the Lohmann paper (Assession number:AJ238799), an HCV cDNA was synthesized by Operon Technologies, Inc. (Alameda, CA), and the full-length replicon was then assembled in plasmid pGem9zf(+) (Promega, Madison, WI) using standard molecular biology techniques. The replicon consists of (i) the HCV 5' UTR fused to the first 12 amino acids of the capsid protein, (ii) the neomycin phosphotransferase gene (neo), (iii) the IRES from encephalomyocarditis virus (EMCV), and (iv) HCV NS3 to NS5B genes and the HCV 3' UTR. Plasmid DNAs were linearized with ScaI and RNA transcripts were synthesized in vitro using the T7 MegaScript transcription kit (Ambion, Austin, TX) according to manufacturer's directions. In vitro transcripts of the cDNA were transfected into the human hepatoma cell line, HUH-7. Selection for cells constitutively expressing the HCV replicon was achieved in the presence of the selectable marker, neomycin (G418). Resulting cell lines were characterized for positive and negative strand RNA production and protein production over time.

*HCV Replicon FRET Assay*

The HCV replicon FRET assay was developed to monitor the inhibitory effects of compounds described in the disclosure on HCV viral replication. HUH-7 cells, constitutively expressing the HCV replicon, were grown in Dulbecco's Modified Eagle Media (DMEM) (Gibco-BRL) containing 10% Fetal calf serum (FCS) (Sigma) and 1 mg/ml G418 (Gibco-BRL). Cells were seeded the night before ($1.5 \times 10^4$ cells/well) in 96-well tissue-culture sterile plates. Compound and no compound controls were prepared in DMEM containing 4% FCS, 1:100 Penicillin/Streptomysin (Gibco-BRL), 1:100 L-glutamine and 5% DMSO in the dilution plate (0.5% DMSO final concentration in the assay). Compound/DMSO mixes were added to the cells and incubated for 4 days at 37°C. After 4 days, cells were first assessed for cytotoxicity using alamar Blue (Trek Diagnotstic Systems) for a $CC_{50}$ reading. The toxicity of compound ($CC_{50}$) was determined by adding $1/10^{th}$ volume of alamar Blue to the media incubating the cells. After 4 h, the fluorescence signal from each well was read, with an excitation wavelength at 530 nm and an emission wavelength of 580 nm, using the Cytofluor Series 4000 (Perspective Biosystems). Plates were then rinsed thoroughly with Phosphate-Buffered Saline (PBS) (3 times 150 $\mu$l). The cells were lysed with 25 $\mu$l of a lysis assay reagent containing an HCV protease substrate (5X cell Luciferase cell culture lysis reagent (Promega #E153A) diluted to 1X with distilled water, NaCl added to 150 mM final, the FRET peptide substrate (as described for the enzyme assay above) diluted to 10 $\mu$M final from a 2 mM stock in 100% DMSO. The plate was then placed into the Cytofluor 4000 instrument which had been set to 340 nm excitation/490 nm emission, automatic mode for 21 cycles and the plate read in a kinetic mode. $EC_{50}$ determinations were carried out as described for the $IC_{50}$ determinations.

*HCV Replicon Luciferase Reporter Assay*

As a secondary assay, $EC_{50}$ determinations from the replicon FRET assay were confirmed in a replicon luciferase reporter assay. Utilization of a replicon luciferase reporter assay was first described by Krieger et al (Krieger N, Lohmann V, and Bartenschlager R, J. Virol. 75(10):4614-4624 (2001)). The replicon construct described for our FRET assay was modified by inserting cDNA encoding a humanized form of the Renilla luciferase gene and a linker sequence fused directly to the 3'-end of the luciferase gene. This insert was introduced into the replicon construct using an Asc1 restriction site located in core, directly upstream of the neomycin marker gene. The adaptive mutation at position 1179 (serine to isoleucine) was also introduced (Blight KJ, Kolykhalov, AA, Rice, CM, Science 290(5498):1972-1974). A stable cell line constitutively expressing this HCV replicon construct was generated as described above. The luciferase reporter assay was set up as described for the HCV replicon FRET assay with the following modifications. Following 4 days in a 37°C/5% $CO_2$ incubator, cells were analyzed for Renilla Luciferase activity using the Promega Dual-Glo Luciferase Assay System. Media (100 $\mu$l) was removed from each well containing cells. To the remaining 50 $\mu$l of media, 50 $\mu$l of Dual-Glo Luciferase Reagent was added, and plates rocked for 10 min to 2 h at room temperature. Dual-Glo Stop & Glo Reagent (50 $\mu$l) was then added to each well, and plates were rocked again for an additional 10 min to 2 h at room temperature. Plates were read on a Packard TopCount NXT using a luminescence program.
The percentage inhibition was calculated using the formula below:

$$\%\ \text{control} = \frac{\text{average luciferase signal in experimental wells (+ compound)}}{\text{average luciferase signal in DMSO control wells (- compound)}}$$

The values were graphed and analyzed using XLfit to obtain the $EC_{50}$ value.

## Claims

1. A compound of Formula (I)

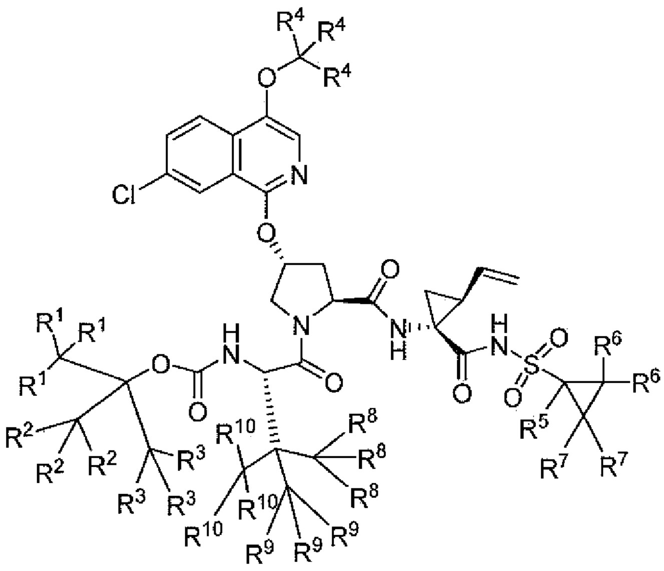

(I),

or a pharmaceutically acceptable salt thereof, wherein
$R^1$, $R^2$, $R^3$, $R^4$, $R^5$, $R^6$, $R^7$, $R^8$, $R^9$, and $R^{10}$ are independently selected from hydrogen and deuterium; provided that at least one is other than hydrogen.

2. A compound of claim 1 wherein each $R^1$ is deuterium.

3. A compound of claim 2 wherein each $R^2$ is deuterium.

4. A compound of claim 3 wherein each $R^3$ is deuterium.

5. A compound of claim 1 wherein each $R^4$ is deuterium.

6. A compound of claim 1 wherein $R^5$ is deuterium.

7. A compound of claim 1 wherein each $R^6$ is deuterium.

8. A compound of claim 7 wherein each $R^7$ is deuterium.

9. A compound of claim 1 wherein each $R^8$ is deuterium.

10. A compound of claim 9 wherein each $R^9$ is deuterium.

11. A compound of claim 10 wherein each $R^{10}$ is deuterium.

12. A compound selected from

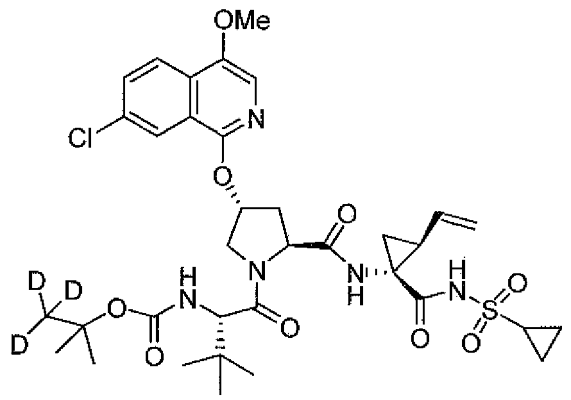

;

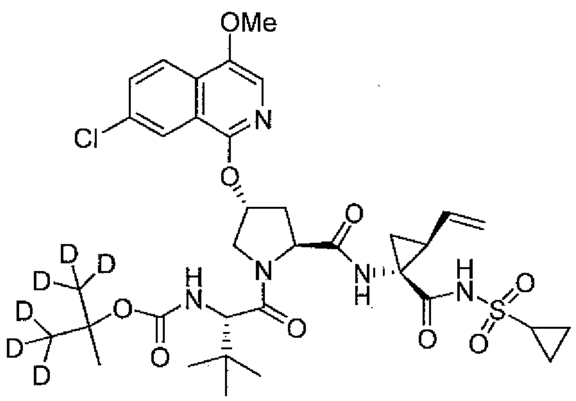

;

and

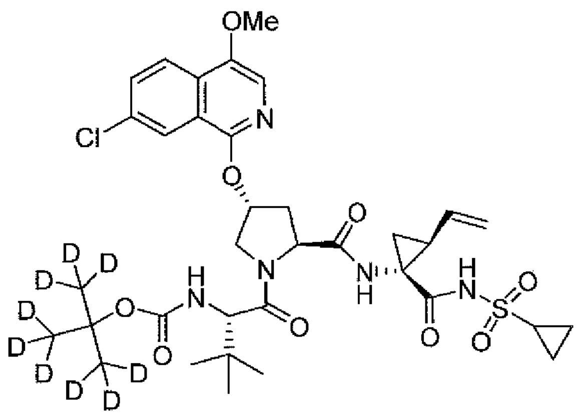

;

or a pharmaceutically acceptable salt thereof.

**13.** A compound selected from

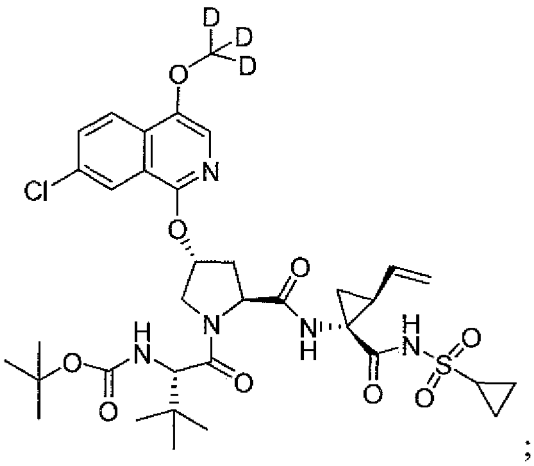
;
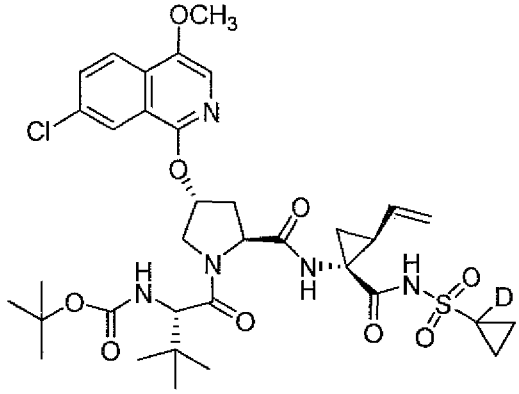
;

and

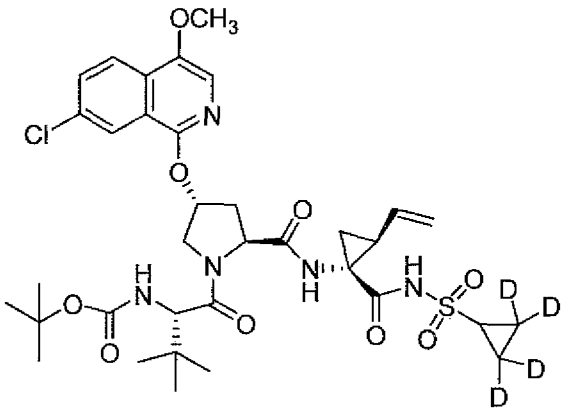
;
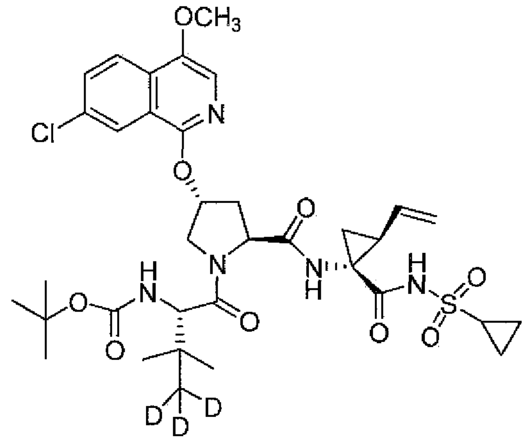
;

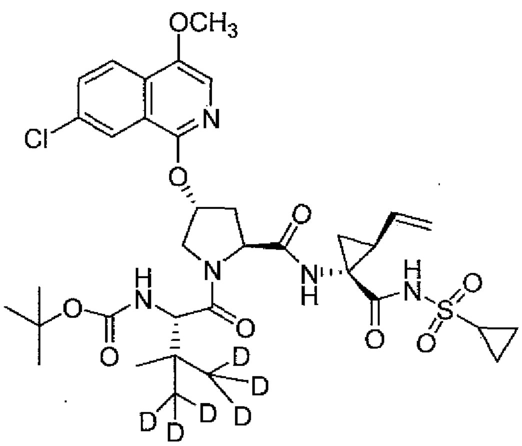
; and
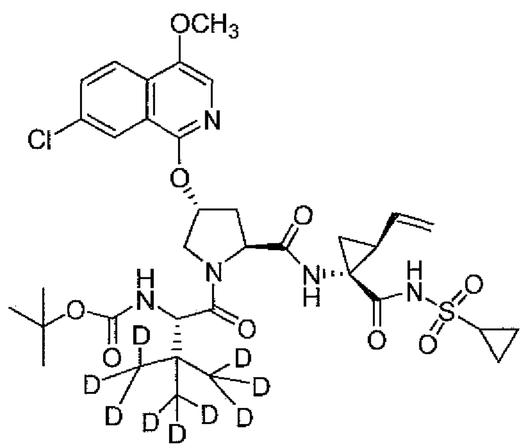
;

or a pharmaceutically acceptable salt thereof.

**14.** A composition comprising the compound of claim 1, or a pharmaceutically acceptable salt thereof, and a pharmaceutically acceptable carrier.

**15.** The composition of claim 14 further comprising at least one additional compound having anti-HCV activity.

**16.** The composition of claim 15 wherein at least one of the additional compounds is an interferon or a ribavirin.

17. The composition of claim 16 wherein the interferon is selected from interferon alpha 2B, pegylated interferon alpha, consensus interferon, interferon alpha 2A, and lymphoblastiod interferon tau.

18. The composition of claim 15 wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

19. The composition of claim 15 wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

20. A compound of claim 1, or a pharmaceutically acceptable salt thereof, for use in a method of treating an HCV infection.

21. The compound, or salt, for use according to claim 20 wherein the compound, or salt, is for being administered prior to, after, or simultaneously with at least one additional compound having anti-HCV activity.

22. The compound, or salt, for use according to claim 21 wherein at least one of the additional compounds is an interferon or a ribavirin.

23. The compound, or salt, for use according to claim 22 wherein the interferon is selected from interferon alpha 2B, pegylated interferon alpha, consensus interferon, interferon alpha 2A, and lymphoblastiod interferon tau.

24. The compound, or salt, for use according to claim 21 wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

25. The compound, or salt, for use according to claim 21 wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

## Patentansprüche

1. Verbindung der Formel (I)

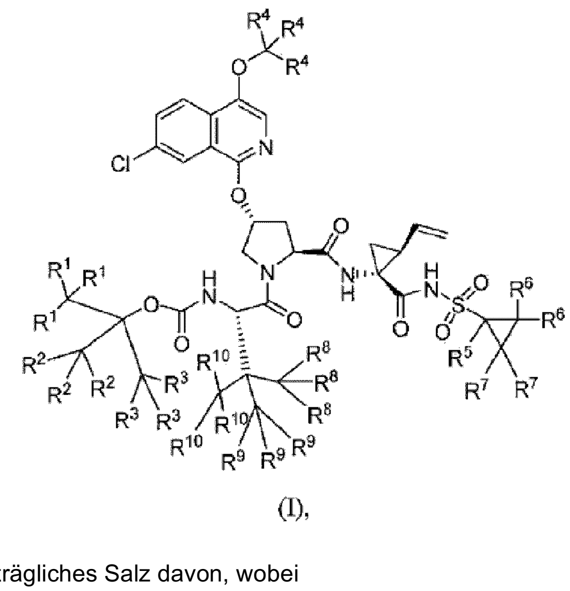

(I),

oder ein pharmazeutisch verträgliches Salz davon, wobei

$R^1$, $R^2$, $R^3$, $R^4$, $R^5$, $R^6$, $R^7$, $R^8$, $R^9$ und $R^{10}$ unabhängig ausgewählt sind aus Wasserstoff und Deuterium; mit der Maßgabe, dass mindestens eines kein Wasserstoff ist.

**2.** Verbindung nach Anspruch 1, wobei jedes $R^1$ Deuterium ist.

**3.** Verbindung nach Anspruch 2, wobei jedes $R^2$ Deuterium ist.

**4.** Verbindung nach Anspruch 3, wobei jedes $R^3$ Deuterium ist.

**5.** Verbindung nach Anspruch 1, wobei jedes $R^4$ Deuterium ist.

**6.** Verbindung nach Anspruch 1, wobei $R^5$ Deuterium ist.

**7.** Verbindung nach Anspruch 1, wobei jedes $R^6$ Deuterium ist.

**8.** Verbindung nach Anspruch 7, wobei jedes $R^7$ Deuterium ist.

**9.** Verbindung nach Anspruch 1, wobei jedes $R^8$ Deuterium ist.

**10.** Verbindung nach Anspruch 9, wobei jedes $R^9$ Deuterium ist.

**11.** Verbindung nach Anspruch 10, wobei jedes $R^{10}$ Deuterium ist.

**12.** Verbindung, ausgewählt aus:

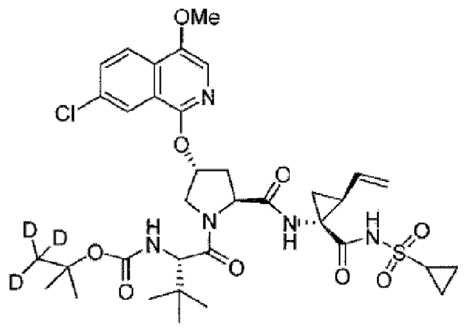

;

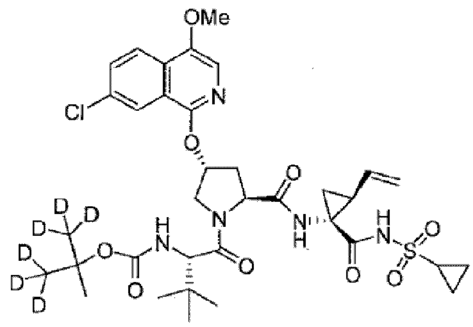

und

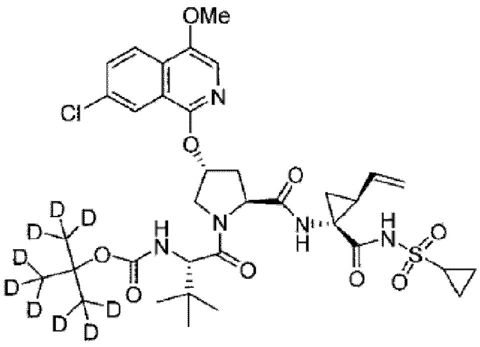

;

oder ein pharmazeutisch verträgliches Salz davon.

**13.** Verbindung, ausgewählt aus:

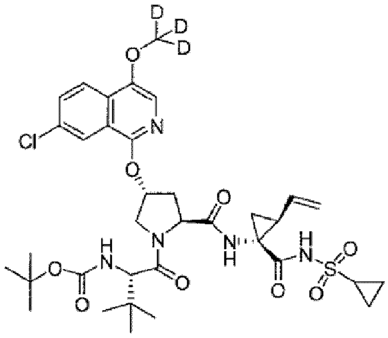

;

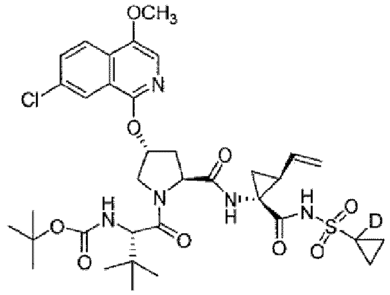

und

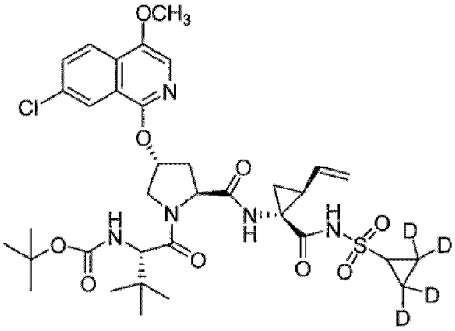

;

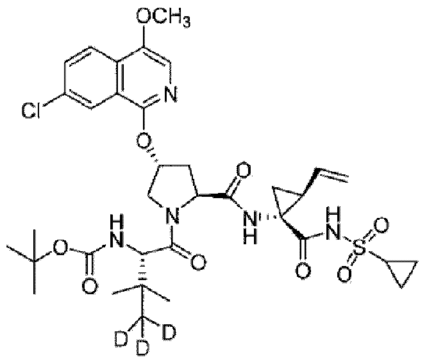

;

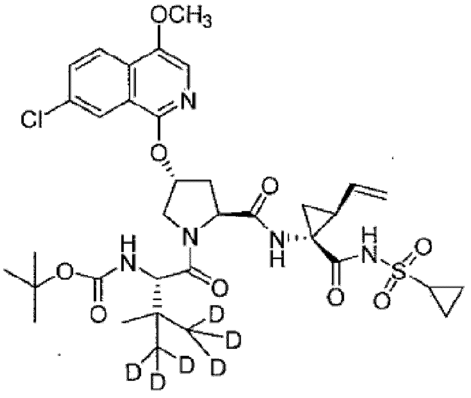

und

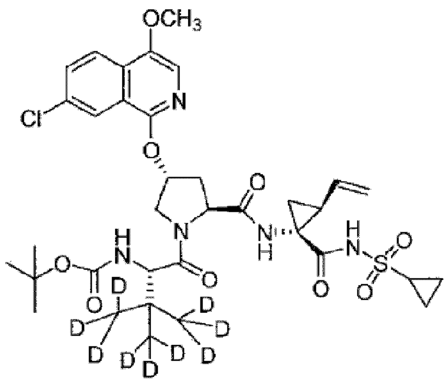

;

oder ein pharmazeutisch verträgliches Salz davon.

**14.** Zusammensetzung, umfassend die Verbindung nach Anspruch 1 oder ein pharmazeutisch verträgliches Salz davon und einen pharmazeutisch verträglichen Träger.

**15.** Zusammensetzung nach Anspruch 14, ferner umfassend mindestens eine zusätzliche Verbindung mit Anti-HCV-Aktivität.

**16.** Zusammensetzung nach Anspruch 15, wobei mindestens eine der zusätzlichen Verbindungen ein Interferon oder ein Ribavirin ist.

**17.** Zusammensetzung nach Anspruch 16, wobei das Interferon ausgewählt ist aus Interferon alpha-2B, pegyliertem Interferon alpha, Konsens-Interferon, Interferon alpha-2A und lymphoblastoidem Interferon tau.

**18.** Zusammensetzung nach Anspruch 15, wobei mindestens eine der zusätzlichen Verbindungen ausgewählt ist aus Interleukin-2, Interleukin-6, Interleukin-12, einer Verbindung, die die Entwicklung einer Typ-1-T-Helferzellenantwort fördert, interferierender RNA, Antisense-RNA, Imiqimod, Ribavirin, einem Inosin-5'-monophospatdehydrogenasehemmer, Amantadin und Rimantadin.

**19.** Zusammensetzung nach Anspruch 15, wobei mindestens eine der zusätzlichen Verbindungen wirksam ist, um die Funktion eines Ziels zu hemmen, das ausgewählt ist aus HCV-Metalloprotease, HCV-Serinprotease, HCV-Polymerase, HCV-Helicase, HCV-NS4B-Protein, HCV-Entry, HCV-Assembly, HCV-Egress, HCV-NS5A-Protein und IMPDH für die Behandlung einer HCV-Infektion.

**20.** Verbindung nach Anspruch 1 oder ein pharmazeutisch verträgliches Salz davon zur Verwendung bei einem Verfahren zur Behandlung einer HCV-Infektion.

**21.** Verbindung oder Salz zur Verwendung nach Anspruch 20, wobei die Verbindung oder das Salz dazu bestimmt sind, vor, nach oder gleichzeitig mit mindestens einer zusätzlichen Verbindung mit Anti-HCV-Aktivität verabreicht zu werden.

**22.** Verbindung oder Salz zur Verwendung nach Anspruch 21, wobei mindestens eine der zusätzlichen Verbindungen ein Interferon oder ein Ribavirin ist.

**23.** Verbindung oder Salz zur Verwendung nach Anspruch 22, wobei das Interferon ausgewählt ist aus Interferon alpha-2B, pegyliertem Interferon alpha, Konsens-Interferon, Interferon alpha-2A und lymphoblastoidem Interferon tau.

**24.** Verbindung oder Salz zur Verwendung nach Anspruch 21, wobei mindestens eine der zusätzlichen Verbindungen ausgewählt ist aus Interleukin-2, Interleukin-6, Interleukin-12, einer Verbindung, die die Entwicklung einer Typ-1-T-Helferzellenantwort fördert, interferierender RNA, Antisense-RNA, Imiqimod, Ribavirin, einem Inosin-5'-monophospatdehydrogenasehemmer, Amantadin und Rimantadin.

**25.** Verbindung oder Salz zur Verwendung nach Anspruch 21, wobei mindestens eine der zusätzlichen Verbindungen wirksam ist, um die Funktion eines Ziels zu hemmen, das ausgewählt ist aus HCV-Metalloprotease, HCV-Serinprotease, HCV-Polymerase, HCV-Helicase, HCV-NS4B-Protein, HCV-Entry, HCV-Assembly, HCV-Egress, HCV-NS5A-Protein und IMPDH, für die Behandlung einer HCV-Infektion.

## Revendications

**1.** Composé de formule (I)

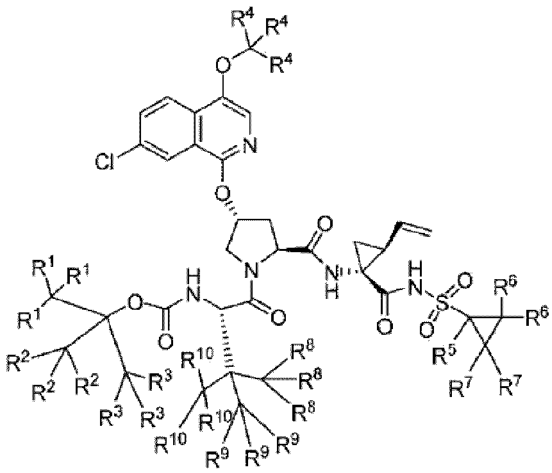

(I),

ou un sel pharmaceutiquement acceptable de celui-ci.
$R^1$, $R^2$, $R^3$, $R^4$, $R^5$, $R^6$, $R^7$, $R^8$, $R^9$ et $R^{10}$ sont indépendamment sélectionnés parmi l'hydrogène et le deutérium; à condition qu'au moins un ne soit pas de l'hydrogène.

**2.** Composé selon la revendication 1, dans lequel chaque $R^1$ est un deutérium.

**3.** Composé selon la revendication 2, dans lequel chaque $R^2$ est un deutérium.

**4.** Composé selon la revendication 3, dans lequel chaque $R^3$ est un deutérium.

**5.** Composé selon la revendication 1, dans lequel chaque $R^4$ est un deutérium.

**6.** Composé selon la revendication 1, dans lequel $R^5$ est un deutérium.

**7.** Composé selon la revendication 1, dans lequel chaque $R^6$ est un deutérium.

**8.** Composé selon la revendication 7, dans lequel chaque $R^7$ est un deutérium.

**9.** Composé selon la revendication 1, dans lequel chaque $R^8$ est un deutérium.

**10.** Composé selon la revendication 9, dans lequel chaque $R^9$ est un deutérium.

**11.** Composé selon la revendication 10, dans lequel chaque $R^{10}$ est un deutérium.

**12.** Composé sélectionné parmi:

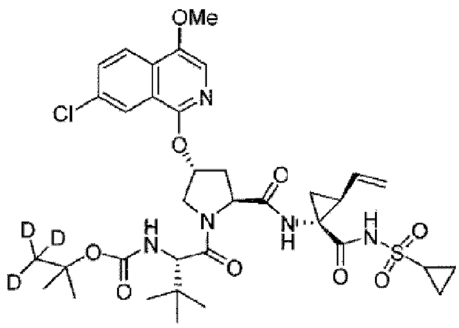

;

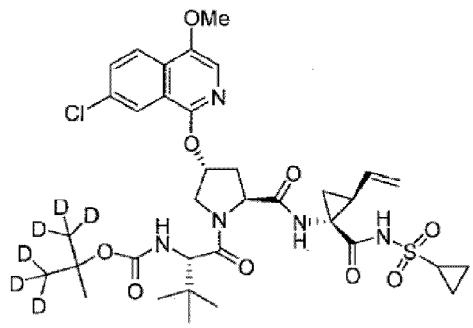

et

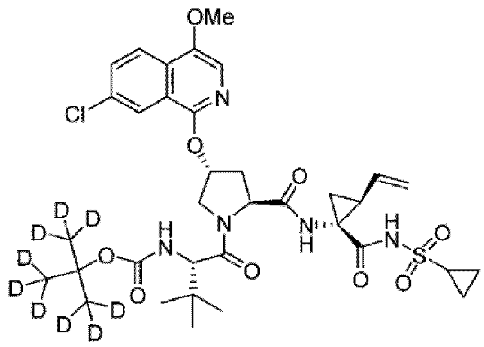

;

ou un sel pharmaceutiquement acceptable de celui-ci.

**13.** Composé sélectionné parmi:

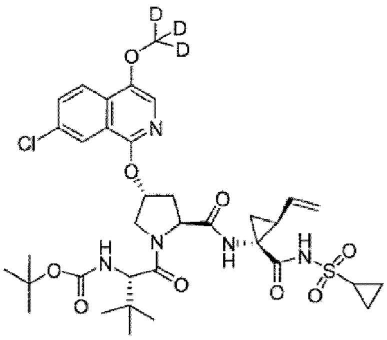

;

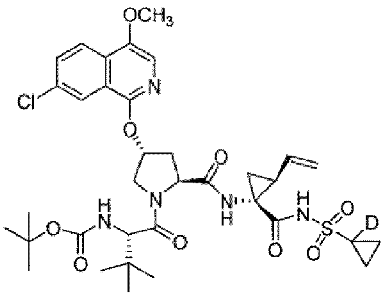

et

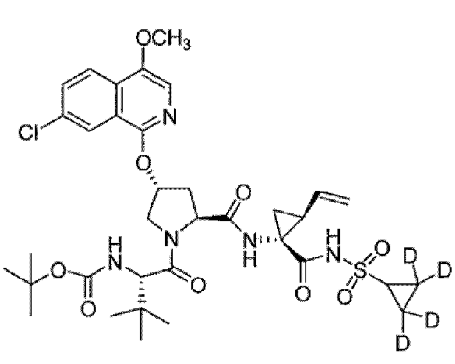

;

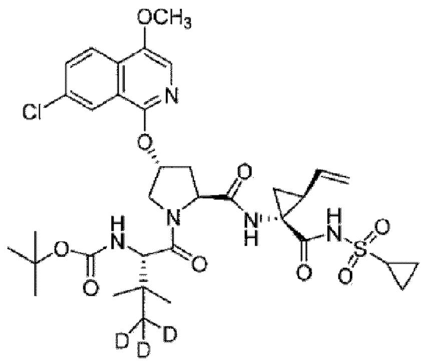

;

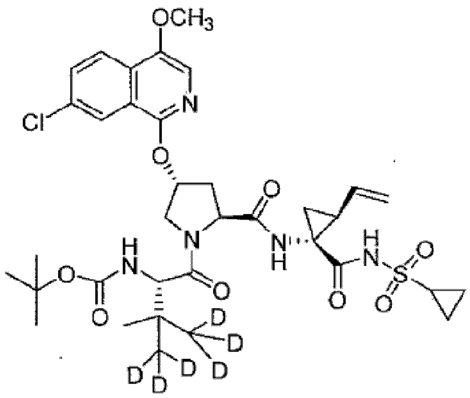 et 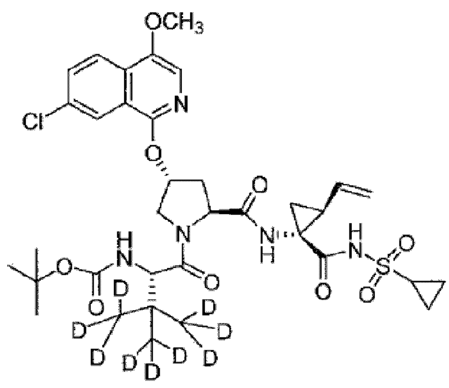 ;

ou un sel pharmaceutiquement acceptable de celui-ci.

14. Composition comprenant le composé selon la revendication 1 ou un sel pharmaceutiquement acceptable de celui-ci, et un excipient pharmaceutiquement acceptable.

15. Composition selon la revendication 14, comprenant en outre au moins un composé supplémentaire ayant une activité anti-VHC.

16. Composition selon la revendication 15, dans laquelle au moins des composés supplémentaires est un interféron ou une ribavirine.

17. Composition selon la revendication 16, dans laquelle l'interféron est sélectionné parmi l'interféron alpha 2B, l'interféron alpha pégylé, l'interféron consensus, l'interféron alpha 2A, et l'interféron lymphoblastoïde tau.

18. Composition selon la revendication 15, dans lequel au moins un des composés supplémentaires est sélectionné parmi l'interleukine 2, l'interleukine 6, l'interleukine 12, un composé qui renforce le développement d'une réponse des cellules T auxiliaires de type 1, un ARN interférent, un ARN anti-sens, de l'imiqimod, de la ribavirine, un inhibiteur de l'inosine 5'-monophosphate déhydrogénase, l'amantadine, et la rimantadine.

19. Composition selon la revendication 15, dans lequel au moins un des composés supplémentaires est efficace pour inhiber la fonction d'une cible sélectionnée parmi la métalloprotéase du VHC, la sérine protéase du VHC, la polymérase du VHC, l'hélicase du VHC, la protéine NS4B du VHC, l'entrée du VHC, l'assemblage du VHC, la sortie du VHC, la protéine NS5A du VHC, et l'IMPDH pour le traitement d'une infection par le VHC.

20. Composition selon la revendication 1 ou un sel pharmaceutiquement acceptable de celle-ci pour une utilisation dans un procédé de traitement d'une infection par le VHC.

21. Composé ou sel pour une utilisation selon la revendication 20 dans lequel le composé ou le sel doit être administré avant, après ou simultanément avec au moins un composé supplémentaire ayant une activité anti-VHC.

22. Composé ou sel pour une utilisation selon la revendication 21, dans lequel au moins un des composés supplémentaires est un interféron ou une ribavirine.

23. Composé ou sel pour une utilisation selon la revendication 22, dans lequel l'interféron est sélectionné parmi l'interféron alpha 2B, l'interféron alpha pégylé, l'interféron consensus, l'interphéron alpha 2A, et l'interféron lymphoblastoïde tau.

24. Composé ou sel pour une utilisation selon la revendication 21, dans lequel au moins un des composés supplémentaires est sélectionné parmi l'interleukine 2, l'interleukine 6, l'interleukine 12, un composé qui renforce le développement d'une réponse des cellules T auxiliaires de type 1, un ARN interférent, un ARN anti-sens, l'imiqimod, la ribavirine, un inhibiteur de l'inosine 5'-monophosphate déhydrogénase, l'amantadine, et la rimantadine.

25. Composé ou sel pour une utilisation selon la revendication 21, dans lequel au moins un des composés supplémentaires est efficace pour inhiber la fonction d'une cible sélectionnée parmi la métalloprotéase du VHC, la sérine

protéase du VHC, la polymérase du VHC, l'hélicase du VHC, la protéine NS4B du VHC, l'entrée du VHC, l'assemblage du VHC, la sortie du VHC, la protéine NS5A du VHC, et l'IMPDH pour le traitement d'une infection par le VHC.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61490665 B
- WO 2005047288 A
- WO 0009543 PCT


### Non-patent literature cited in the description


- Remington's Pharmaceutical Sciences. Mack Publishing Company, 1999
- **BERGER A. ; SCHECHTER I.** Transactions of the Royal Society London series. 1970, vol. B257, 249-264
- *Pharmaceutical Research,* 1986, vol. 3 (6), 318
- **P SIMMONDS ; KA ROSE ; S GRAHAM ; SW CHAN ; F MCOMISH ; BC DOW ; EA FOLLETT ; PL YAP ; H MARSDEN.** *J. Clin. Microbiol.,* 1993, vol. 31 (6), 1493-1503
- **YANAGI,M. ; PURCELL,R.H. ; EMERSON,S.U. ; BUKH.** *J. Proc. Natl. Acad. Sci. U.S.A.,* 1997, vol. 94 (16), 8738-8743
- **YANAGI,M. ; ST CLAIRE,M. ; SHAPIRO,M. ; EMERSON,S.U. ; PURCELL,R.H. ; BUKH, J.** *Virology,* 1998, vol. 244 (1), 161-172
- **GALLINARI P ; PAOLINI C ; BRENNAN D ; NARDI C ; STEINKUHLER C ; DE FRANCESCO R.** *Biochemistry,* 1999, vol. 38 (17), 5620-32
- **GALLINARI P ; BRENNAN D ; NARDI C ; BRUNETTI M ; TOMEI L ; STEINKUHLER C ; DE FRANCESCO R.** *J Virol.,* 1998, vol. 72 (8), 6758-69
- **TALIANI et al.** *Anal. Biochem.,* 1996, vol. 240 (2), 60-67
- **LOHMANN V ; KORNER F ; KOCH J ; HERIAN U ; THEILMANN L ; BARTENSCHLAGER R.** *Science,* 1999, vol. 285 (5424), 110-3
- **KRIEGER N ; LOHMANN V ; BARTENSCHLAGER R.** *J. Virol.,* 2001, vol. 75 (10), 4614-4624
- **BLIGHT KJ ; KOLYKHALOV, AA ; RICE, CM.** *Science,* vol. 290 (5498), 1972-1974